(19) 

(11) **EP 4 796 092 A1**

(12) **EUROPEAN PATENT APPLICATION**
published in accordance with Art. 153(4) EPC


(43) Date of publication:
**26.08.2026 Bulletin 2026/35**

(21) Application number: **24911976.9**

(22) Date of filing: **25.10.2024**

(51) International Patent Classification (IPC):
***A61F 13/535*** *(2006.01)* ***A61F 13/53*** *(2006.01)*
***A61F 13/56*** *(2006.01)* ***A61F 13/511*** *(2006.01)*
***A61F 13/536*** *(2006.01)* ***A61F 13/539*** *(2006.01)*

(52) Cooperative Patent Classification (CPC):
**A61F 13/472; A61F 13/475; A61F 13/511; A61F 13/53; A61F 13/535; A61F 13/536; A61F 13/537; A61F 13/539; A61F 13/56; A61F 13/84**

(86) International application number:
**PCT/JP2024/038160**

(87) International publication number:
**WO 2025/142083 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.12.2023 JP 2023222358**



(71) Applicant: **Unicharm Corporation**
**Ehime 799-0111 (JP)**

(72) Inventors:
- **SAITO, Haruna**
  **Kanonji-shi, Kagawa 769-1602 (JP)**
- **ISHIKAWA, Sei**
  **Kanonji-shi, Kagawa 769-1602 (JP)**
- **UDA, Masashi**
  **Kanonji-shi, Kagawa 769-1602 (JP)**

(74) Representative: **Dolleymores**
**9 Rickmansworth Road**
**Watford, Hertfordshire WD18 0JU (GB)**


(54) **ABSORBENT ARTICLE**


(57) An absorbent (1) comprising an absorbent core (11) having a longitudinal direction, width direction, and thickness direction orthogonal to each other and including pulverized fibers is characterized in that the pulverized fibers include hardwood fibers composed of hardwood, the absorbent core (11) is provided with an oil agent (Y) that promotes diffusion of excretion, when divided into three equal parts in either the longitudinal direction or the width direction, the absorbent core (11) includes one side portion, a central portion, and another side portion in order from one side, and the basis weight of the oil agent (Y) provided in the central portion is higher than the basis weight of the oil agent provided in the one side portion.


EP 4 796 092 A1

WIDTH DIRECTION
(ONE SIDE)
(OTHER SIDE)
1
C
10
Ta
1W
Y21
1W
33
Y21u
(FRONT SIDE) ONE SIDE
Y11,R1
D
D
Y11k
33
LONGITUDINAL DIRECTION
CL
Tb
T11
OTHER SIDE (BACK SIDE)
EL
EL
11d
23
23
Tc
21
C
Wa
Wb
Wc
W11

FIG. 8

## Description

FIELD

The present invention relates to an absorbent article.

BACKGROUND

Absorbent articles such as sanitary napkins that absorb excrement are known. In the case where excrement such as menstrual blood is discharged during wear, it is preferable that the absorbent article quickly absorbs the discharged excrement. For example, Patent Literature 1 discloses a sanitary napkin that includes a top sheet and an absorbent body, the top sheet coming into contact with the wearer's skin, the absorbent body being located on the non-skin side with respect to the top sheet. In this sanitary napkin, an agent for imparting blood slipperiness is provided in the top sheet for the purpose of promoting absorption of excrement such as menstrual blood with high viscosity during wear. This facilitates the transfer of the discharged excrement from the top sheet to the absorbent body, thereby reducing perception of stickiness on the skin-side surface of the top sheet.

[CITATION LIST]

[PATENT LITERATURE]

Patent Literature 1: Japanese Patent Application Publication No. 2013-179982

SUMMARY

[TECHNICAL PROBLEM]

However, the sanitary napkin disclosed in Patent Literature 1 promotes absorption of excrement while at the same time excessively promoting diffusion of excrement in the sanitary napkin, which may lead to leakage of excrement from the sanitary napkin.

The present invention was achieved in light of problems such as that described above, and an object of the present invention is to provide an absorbent article that suppresses diffusion of excrement in a one-side portion of the absorbent article in a longitudinal direction or a width direction, thereby reducing a risk that excrement leaks to the outside beyond a one-side end of the absorbent article in the longitudinal direction or the width direction.

[SOLUTION TO PROBLEM]

A main aspect of the present invention for achieving the above-described aspect is an absorbent article having a longitudinal direction, a width direction, and a thickness direction that are orthogonal to one another, the absorbent article including: an absorbent core that has pulverized fibers, the pulverized fibers including hardwood fibers that are made from hardwood, an oil agent being provided in the absorbent core, the oil agent promoting diffusion of excrement, in either the longitudinal direction or the width direction, when the absorbent core is divided into three equal portions, the three equal portions being a one-side portion, a central portion, and an other-side portion, in order from one side, a basis weight of the oil agent provided in the central portion being higher than a basis weight of the oil agent provided in the one-side portion. Features of the present invention other than the above will become clear by reading the description of the present specification with reference to the accompanying drawings.

[Advantageous Effects of Invention]

According to the present invention, it is possible to suppress diffusion of excrement in a one-side portion of the absorbent article in a longitudinal direction or a width direction, thereby reducing a risk that excrement leaks to the outside beyond a one-side end of the absorbent article in the longitudinal direction or the width direction.

[Brief Description of the Drawings]

FIG. 1 is a plan view of a sanitary napkin 1 as viewed from the skin side.

FIG. 2 is a plan view of the sanitary napkin 1 as viewed from the non-skin side.
FIG. 3 is a schematic cross-sectional view taken along line A-A in FIG. 1.
FIG. 4A is a plan view of an absorbent core 11.
FIG. 4B is a schematic cross-sectional view taken along line J-J in FIG. 4A.
FIG. 5 is an enlarged schematic cross-sectional view taken along line K-K in FIG. 4A.
FIG. 6 is an enlarged view of an absorbent body 10 in FIG. 3.
FIG. 7 is a plan view of the absorbent body 10 as viewed from the skin side.
FIG. 8 is a schematic plan view illustrating a region in which an oil agent Y is provided.
FIG. 9 is a cross-sectional schematic view illustrating the region in which the oil agent Y is provided.
FIG. 10 is a plan view of a sanitary napkin 100 as viewed from the non-skin side.

DESCRIPTION OF EMBODIMENTS

At least the following matters will become clear with the description of this specification and the attached drawings.

Aspect 1

An absorbent article having a longitudinal direction, a width direction, and a thickness direction that are orthogonal to one another, the absorbent article including: an absorbent core that has pulverized fibers, the pulverized fibers including hardwood fibers that are made from hardwood, an oil agent being provided in the absorbent core, the oil agent promoting diffusion of excrement, in either the longitudinal direction or the width direction, when the absorbent core is divided into three equal portions, the three equal portions being a one-side portion, a central portion, and an other-side portion, in order from one side, a basis weight of the oil agent provided in the central portion being higher than a basis weight of the oil agent provided in the one-side portion.

According to Aspect 1, in either the longitudinal direction or the width direction, the basis weight of the oil agent in the central portion is higher than the basis weight of the oil agent in the one-side portion, thereby facilitating diffusion of excrement in the central portion due to the oil agent. On the other hand, in the one-side portion of the absorbent core, the absorbent core includes hardwood fibers, and the basis weight of the oil agent in the one-side portion is lower than the basis weight of the oil agent in the central portion, thereby suppressing diffusion of excrement in the one-side portion of the absorbent core and the absorbent article in the longitudinal direction or the width direction. This can reduce the risk that excrement leaks outward from the one-side end of the absorbent article in the longitudinal or the width direction. In addition, during wear, the wearer visually recognizes the state in which excrement is more likely to diffuse in the central portion of the absorbent core and less likely to diffuse in the one-side portion in the longitudinal direction or the width direction, thereby making the wearer’s concern that the excrement may leak from the absorbent article likely to be alleviated.

Aspect 2

The absorbent article according to Aspect 1, wherein the pulverized fibers include softwood fibers that are made from softwood, and a total weight of the hardwood fibers is greater than a total weight of the softwood fibers.

According to Aspect 2, the absorbent core has a large amount of hardwood fibers with a short average fiber length, thereby making it easier, due to the oil agent, to promote absorption of excrement in the central portion of the absorbent core while making it easier to suppress diffusion of excrement in the one-side portion of the absorbent core in the longitudinal direction or the width direction.

Aspect 3

The absorbent article according to Aspect 1 or 2, wherein the absorbent article further comprises a top-face sheet capable of coming into contact with a skin of a wearer, the oil agent is provided in the top-face sheet, and as viewed in the thickness direction, in the top-face sheet, a basis weight of the oil agent provided in a portion overlapping the central portion is higher than a basis weight of the oil agent provided in a portion overlapping the one-side portion.

According to Aspect 3, the oil agent facilitates absorption of excrement in the central portion of the absorbent core while facilitating suppression of diffusion of excrement in the one-side portion of the absorbent core in the longitudinal direction or the width direction.

Aspect 4

The absorbent article according to any one of Aspects 1 to 3, wherein the oil agent is an amphiphilic substance.

According to Aspect 4, the surface tension of excrement can be more readily changed, thereby facilitating diffusion of excrement in the absorbent core.

Aspect 5

The absorbent article according to any one of Aspects 1 to 4, wherein a core-wrapping sheet is provided on a skin side with respect to the absorbent core, and an adhesive is not provided between a skin-side surface of the absorbent core and a non-skin-side surface of the core-wrapping sheet.

According to Aspect 5, on the skin-side surface of the absorbent core, the compatibility between the oil agent and the adhesive causes the oil agent to diffuse on the skin-side surface of the core-wrapping sheet, whereby excrement is absorbed from the core-wrapping sheet into the absorbent core and the risk of excessive diffusion of excrement in the absorbent core is reduced, thereby reducing the risk that excrement leaks to the outside of the absorbent article.

Aspect 6

The absorbent article according to any one of Aspects 1 to 5, wherein a core-wrapping sheet is provided on a non-skin side with respect to the absorbent core, and an adhesive is not provided between a non-skin-side surface of the absorbent core and a skin-side surface of the core-wrapping sheet.

According to Aspect 6, on the non-skin-side surface of the absorbent core, the compatibility between the oil agent and the adhesive causes the oil agent to diffuse on the skin-side surface of the core-wrapping sheet, whereby excrement is absorbed from the core-wrapping sheet into the absorbent core and the risk of excessive diffusion of excrement in the absorbent core is reduced, thereby reducing the risk that excrement leaks to the outside of the absorbent article.

Aspect 7

The absorbent article according to any one of Aspects 1 to 6, wherein a post-processing tab or a post-processing tape is provided on a non-skin-side surface of the absorbent article, and as viewed in the thickness direction, the oil agent does not overlap the post-processing tab or the post-processing tape.

According to Aspect 7, since the shape of the absorbent core in which excrement has diffused due to the oil agent is likely to collapse, the post-processing tab or the post-processing tape is provided, as viewed in the thickness direction, at a position that does not overlap the oil agent, which is a portion where the shape of the absorbent core is less likely to collapse, thereby allowing the absorbent core to be easily rolled while preventing the shape of the absorbent core from collapsing when the absorbent article is rolled up for disposal after use.

Aspect 8

The absorbent article according to any one of Aspects 1 to 7, wherein a non-skin-side sheet is provided farthest on a non-skin side of the absorbent article, an adhesive portion is provided on a non-skin-side surface of the non-skin-side sheet, as viewed in the thickness direction, there is an overlapping region and a non-overlapping region, the overlapping region being a region in which a portion provided with the oil agent overlaps the adhesive portion, the non-overlapping region being a region in which a portion not provided with the oil agent overlaps the adhesive portion, and an adhesive strength of the adhesive portion in the overlapping region is lower than an adhesive strength of the adhesive portion in the non-overlapping region.

The adhesive portion that is provided on the non-skin side of the absorbent article is provided for fixing the clothing of the wearer during wear. According to Aspect 8, it is possible to reduce the risk that the adhesive strength of the adhesive portion is excessively high, and, when removing, from the clothing, the absorbent article including the overlapping region that has become wrinkled after absorbing a large amount of body fluid, the absorbent article can be removed with a small force. This makes the shape of the absorbent core in the overlapping region less likely to collapse, thereby improving disposability.

Aspect 9

The absorbent article according to any one of Aspects 1 to 8, wherein the absorbent core includes a core-wrapping sheet on a skin side of the absorbent core, and an average fiber length of fibers of the core-wrapping sheet is equal to or greater than an average fiber length of the pulverized fibers of the absorbent core.

According to Aspect 9, during wear, absorption and diffusion of the excrement in the core-wrapping sheet are promoted, thereby making it easier to promote penetration of the excrement into the absorbent core located on the non-skin side.

Aspect 10

The absorbent article according to any one of Aspects 1 to 9, wherein in the longitudinal direction and the width direction, a basis weight of the oil agent provided in the central portion is higher than a basis weight of the oil agent provided in the one-side portion.

According to Aspect 10, it is possible to promote diffusion of excrement in the central portion of the absorbent core in the longitudinal direction and the width direction, while reducing a risk that excrement leaks from the outer side on the one side of the absorbent article in the longitudinal direction and the width direction.

Aspect 11

The absorbent article according to Aspect 10, wherein the oil agent is not provided in an outer end portion of the one-side portion.

According to Aspect 11, it is possible to more readily suppress diffusion of excrement in the outer end portion of the one-side portion of the absorbent core in the longitudinal direction and the width direction, thereby reducing a risk that excrement leaks from the outer side on the one side of the absorbent article in the longitudinal direction and the width direction.

Aspect 12

The absorbent article according to any one of Aspects 1 to 11, wherein the absorbent article further comprises a top-face sheet capable of coming into contact with a skin of a wearer, the oil agent is provided in the top-face sheet, a widthwise length of the top-face sheet is longer than a widthwise length of the absorbent core, the absorbent core has a core-oil-agent-present region and a core-oil-agent-absent region, the core-oil-agent-present region being a region in which the oil agent is provided, the core-oil-agent-absent region being a region in which the oil agent is not provided, the top-face sheet has a surface-oil-agent-present region and a surface-oil-agent-absent region, the surface-oil-agent-present region being a region in which the oil agent is provided, the surface-oil-agent-absent region being a region in which the oil agent is not provided, in the thickness direction, there are a first region, a second region, and a third region, the first region being a region in which the core-oil-agent-present region and the surface-oil-agent-present region overlap, the second region being a region in which the core-oil-agent-absent region and the surface-oil-agent-present region overlap, the third region being a region in which the surface-oil-agent-absent region and the absorbent core do not overlap, and the first region, the second region, and the third region are arranged in order from an inner side to an outer side in the width direction.

According to Aspect 12, in the absorbent article, diffusion of excrement is promoted in the first region, and diffusion of excrement in the second region and the third region is more readily suppressed. Thus, it is possible to reduce the risk that excrement leaks to the outside of the absorbent article.

Aspect 13

The absorbent article according to Aspect 12, wherein the core-oil-agent-present region has, in the first region, a core-oil-agent-high-basis-weight region in which a basis weight of the oil agent is higher than that in a surrounding region, the surface-oil-agent-present region has, in the first region, a surface-oil-agent-low-basis-weight region in which the basis weight of the oil agent is lower than that in a surrounding region, and as viewed in the thickness direction, the first region has, in a central portion in both the width direction and the longitudinal direction, a portion in which the core-oil-agent-high-basis-weight region and the surface-oil-agent-low-basis-weight region overlap.

According to Aspect 13, during wear, the surface-oil-agent-low-basis-weight region makes it possible to reduce the risk that the oil agent causes irritation or adverse effects to the wearer's excretion opening while, in the surface-oil-agent-present region of the first region, making it easier to promote penetration and diffusion of excrement in the top-face sheet. In addition, as viewed in the thickness direction, by providing the core-oil-agent-high-basis-weight region that overlaps the surface-oil-agent-low-basis-weight region, a portion in which the core-oil-agent-high-basis-weight region and the surface-oil-agent-low-basis-weight region overlap as viewed in the thickness direction makes it easier to transfer excrement from the top-face sheet to the absorbent core, thereby reducing the risk that excrement diffuses on the skin-side surface of the absorbent article and leaks out.

Aspect 14

The absorbent article according to any one of Aspects 1 to 13, wherein the absorbent article further comprises a top-face sheet capable of coming into contact with a skin of a wearer, the oil agent is provided in the top-face sheet, a widthwise length of the top-face sheet is longer than a widthwise length of the absorbent core, and the oil agent is not provided in a region located outside the absorbent core in the width direction.
According to Aspect 14, diffusion of excrement in the widthwise end portions of the absorbent core is more readily suppressed, thereby reducing the risk that the excrement leaks to the outside of the absorbent article.

Aspect 15

The absorbent article according to any one of Aspects 1 to 14, wherein the absorbent article further comprises a compressed portion in which the absorbent core is compressed in the thickness direction, the compressed portion includes a back compressed portion on a back side with respect to a center of the absorbent article in the longitudinal direction, and a back end of the oil agent is provided on a front side with respect to a front end of the back compressed portion.
According to Aspect 15, since the back compressed portion having a high fiber density tends to promote diffusion due to capillary action, by providing the oil agent on the front side with respect to the front end of the back compressed portion, it becomes easier to suppress diffusion of excrement in a back portion of the absorbent core, thereby reducing the risk that excrement leaks to the outside of the absorbent article.

Aspect 16

The absorbent article according to any one of Aspects 1 to 15, wherein a weight per unit volume of the oil agent provided on a skin side with respect to a center of the absorbent core in the thickness direction is greater than a weight per unit volume of the oil agent provided on a non-skin side with respect to the center of the absorbent core in the thickness direction.
According to Aspect 16, excrement absorbed from the skin side is more readily absorbed toward the non-skin side.

Aspect 17

The absorbent article according to any one of Aspects 1 to 16, wherein the absorbent article further comprises a fold portion extending in the width direction, and in the longitudinal direction, the oil agent is provided so as to span the fold portion.
According to Aspect 17, since the fold portion tends to have a high density of the absorbent core and therefore tends to promote diffusion of excrement in the width direction; however, by providing the oil agent so as to span the fold portion in the longitudinal direction, diffusion of excrement in the longitudinal direction can be more readily promoted, thereby reducing the risk that excrement leaks to the outside of the absorbent article in the width direction.

Embodiment

The following describes an absorbent article according to an embodiment, using a sanitary napkin as an example. However, the sanitary absorbent article is not limited to the sanitary napkin, and may be underpants-shaped napkins, disposable underpants-shaped diapers, tape-type disposable diapers, and absorbent pads.

Configuration of Sanitary Napkin 1 of Present Embodiment

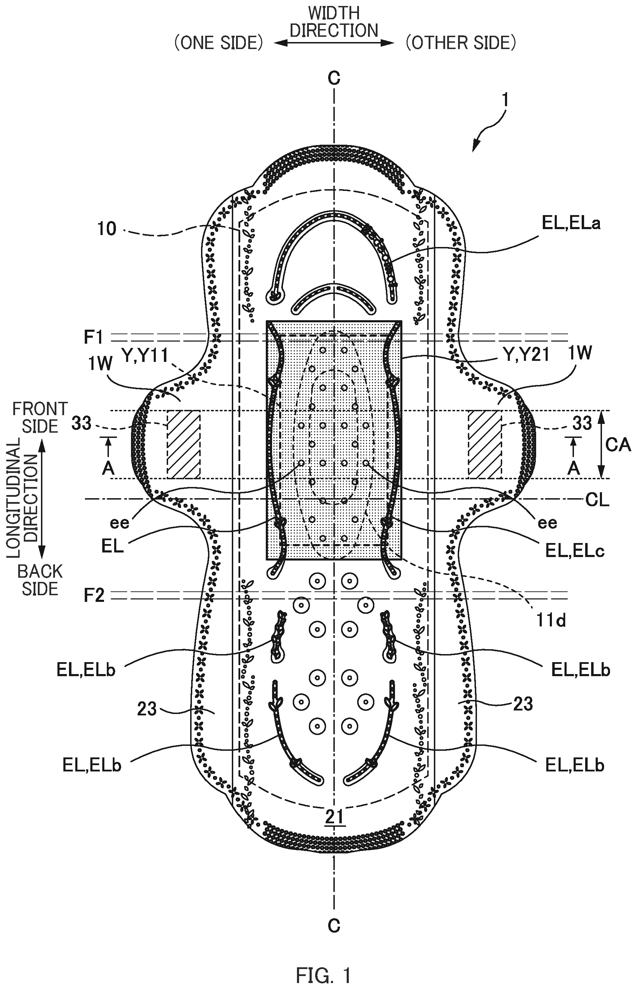

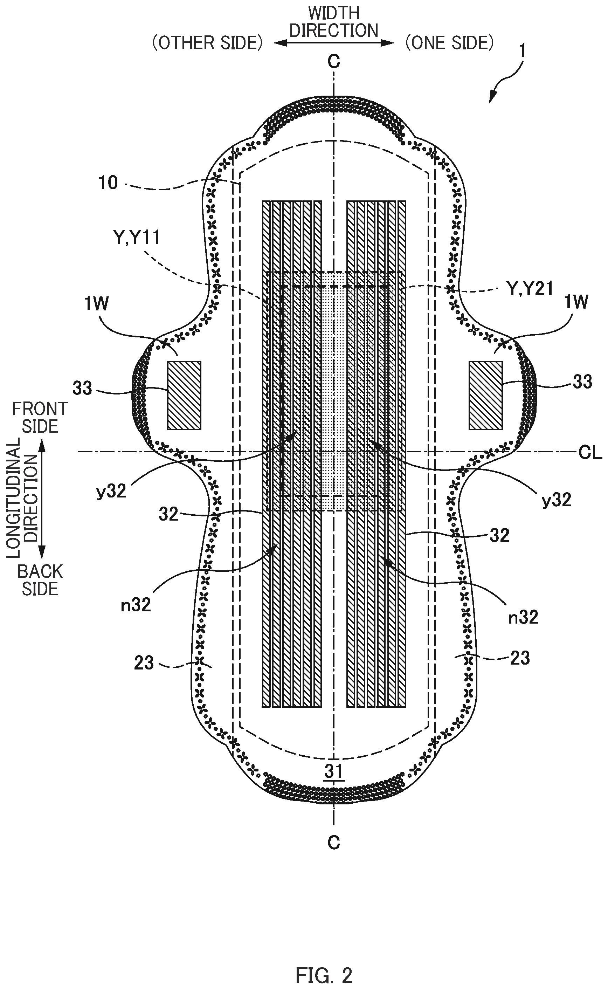

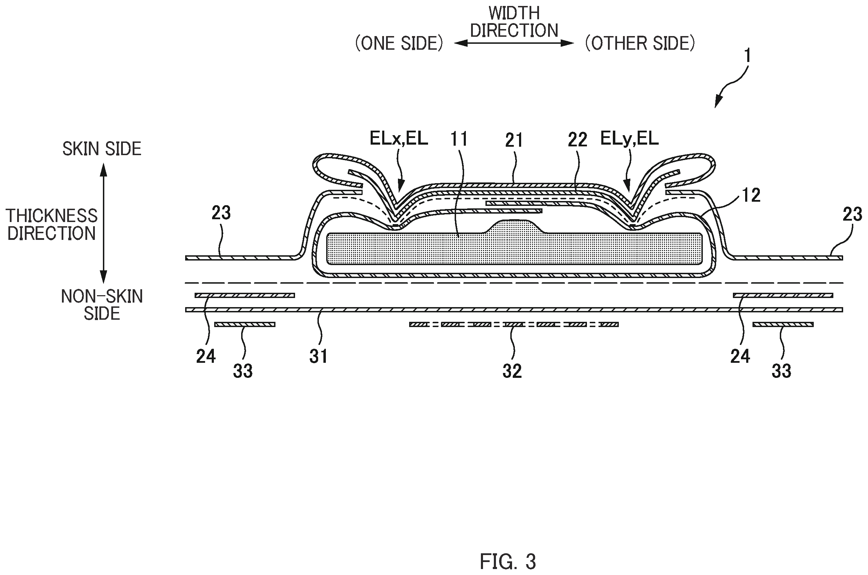

FIG. 1 is a plan view of a sanitary napkin 1 as viewed from the skin side. FIG. 2 is a plan view of the sanitary napkin 1 as viewed from the non-skin side. FIG. 3 is a schematic cross-sectional view taken along line A-A in FIG. 1. The sanitary napkin 1 has a longitudinal direction, a width direction, and a thickness direction. In the longitudinal direction, the side that comes into contact with the wearer's lower stomach region is referred to as the front side, and the side that comes into contact with the wearer's buttocks region is referred to as the back side. In the thickness direction, the side that comes into contact with the wearer's skin is referred to as the skin side, and the side opposite thereto is referred to as the non-skin side. A center line C-C shown in FIG. 1 and the like represents the center of the sanitary napkin 1 in the width direction, and a center line CL represents the center of the sanitary napkin 1 in the longitudinal direction.
The cross-sectional views in FIG. 3 and the like schematically illustrate the sanitary napkin 1, and the dimensions are not necessarily accurate.

The sanitary napkin 1 includes an absorbent body 10, a top sheet 21, a pair of side sheets 23, and a back sheet 31. A sheet-shaped second sheet 22 may be provided on the skin side with respect to the absorbent body 10, and on the non-skin side with respect to the top sheet 21. The materials adjacent to one another in the thickness direction are joined together by an adhesive such as a hot-melt adhesive.

The sanitary napkin 1 has, in its central portion in the longitudinal direction, a pair of wing portions 1W respectively extending outward on both widthwise sides. The wing portions 1W are mainly formed by the side sheet 23 and the back sheet 31. The sanitary napkin 1 does not necessarily have the wing portions 1W.

The top sheet 21 is a liquid-permeable sheet that is located on the skin side with respect to the absorbent body 10. The top sheet 21 is a top-face sheet that is located on the skin side with respect to the absorbent body 10 and can come into contact with the wearer's skin. Examples of the top sheet 21 include soft sheets such as nonwoven fabric sheets made of synthetic fibers such as polyethylene (PE) and polypropylene (PP) (for example, air-through nonwoven fabric sheets or spunbond nonwoven fabric sheets), synthetic resin films having through-holes, and nonwoven fabric sheets made of cotton. The top sheet 21 of the present embodiment is formed of a liquid-permeable sheet that is made of polyethylene terephthalate (PET) and polyethylene (PE), or made of air-through nonwoven fabric or spunlace nonwoven fabric containing water-retaining fibers such as cotton or rayon.

The pair of side sheets 23 is provided on the non-skin side with respect to the top sheet 21 and the second sheet 22, and on the skin side with respect to the absorbent body 10, and extends outward from both widthwise side portions of the top sheet 21 and the like, respectively. Examples of the side sheet 23 include a soft liquid-permeable nonwoven fabric sheet similar to the top sheet 21, a hydrophobic nonwoven fabric sheet, and the like. Furthermore, a reinforcing sheet 24 that reinforces the strength of the side sheet 23 is provided between the side sheet 23 and the back sheet 31 in the thickness direction, at a position outside the top sheet 21, the second sheet 22, and the absorbent body 10 in the width direction. As the reinforcing sheet 24, a sheet member similar to the back sheet 31 or a sheet member made of paper, SMS, or the like can be used, for example.

The back sheet (non-skin-side sheet) 31 is located on the non-skin side with respect to the absorbent body 10 and provided farthest on the non-skin side of the sanitary napkin 1. Examples of the back sheet 31 include a liquidimpermeable sheet such as a polyethylene (PE) resin film. The shape of the back sheet 31 is substantially the same as the external shape of the sanitary napkin 1.

A plurality of main-body adhesive portions (adhesive portions) 32 are provided on the non-skin-side surface of the back sheet 31. The main-body adhesive portions 32 of the present embodiment are regions in which a plurality of strip-shaped hot-melt adhesives are applied along the longitudinal direction. When the sanitary napkin 1 is worn, the main-body adhesive portions 32 are attached to an inner side of a crotch portion of underwear (garment), thereby preventing the position of the sanitary napkin 1 from shifting relative to the wearer's body.

The wing portions 1W each have a wing adhesive portion 33 on the non-skin surface side of the back sheet 31. The wing adhesive portion 33 of the present embodiment is a region in which a hot-melt adhesive is applied in a rectangular shape. When the sanitary napkin 1 is worn, the wing portions 1W are folded back toward the non-skin-side surface of the underwear, and the wing adhesive portions 33 are attached to the non-skin-side surface at the crotch portion of the underwear, thereby fixing the sanitary napkin 1 to the underwear.

The position overlapping the wing adhesive portions 33 in the longitudinal direction (see FIG. 1) is a crotch region CA. The crotch region CA is a region that, in a put-on state, comes into contact with the wearer's crotch portion in the longitudinal direction, and also comes into contact with a vaginal opening from which the wearer's excrement is discharged.

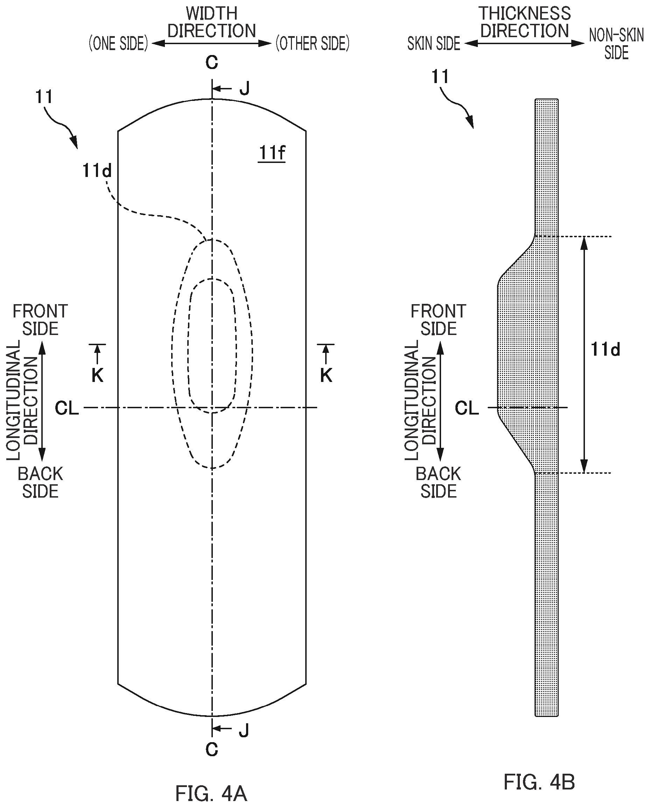

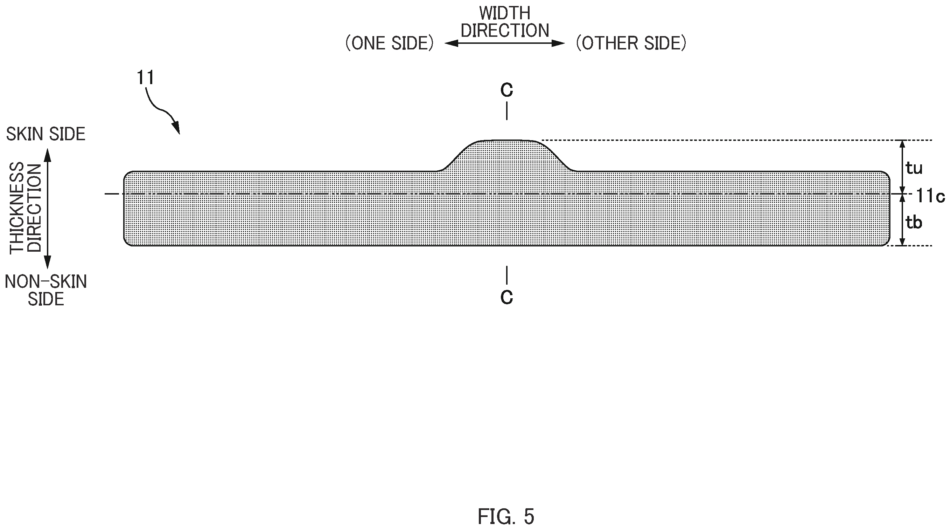

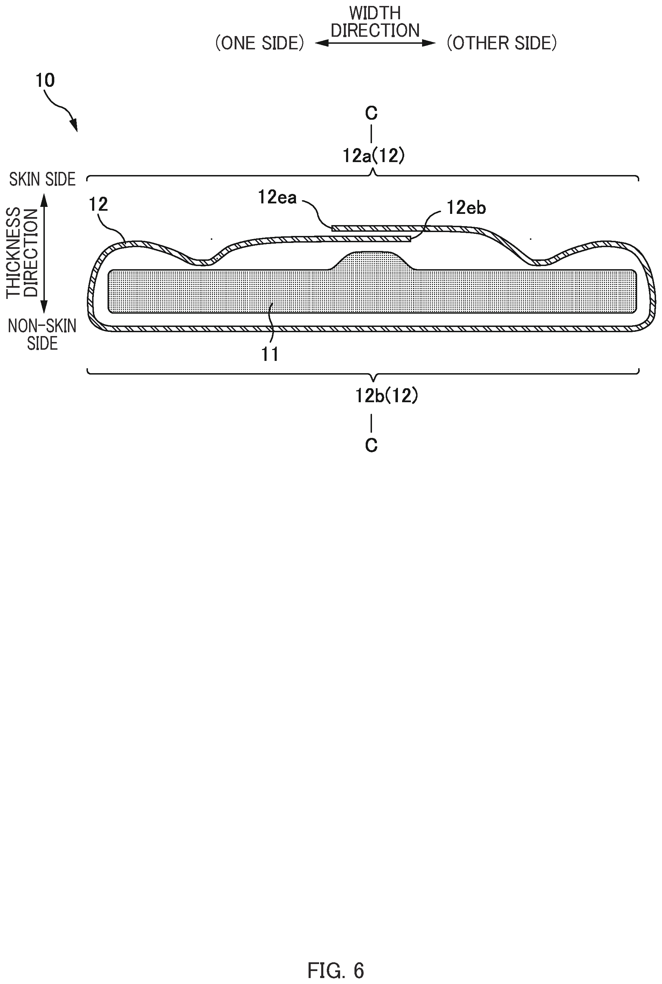

The absorbent body 10 includes an absorbent core 11 and a core-wrapping sheet 12. The absorbent core 11 is formed by shaping liquid-absorbent fibers into a predetermined shape, and has pulverized fibers as the liquid-absorbent fibers. The pulverized fibers include hardwood fibers made from hardwood. The absorbent core 11 of the present embodiment has pulverized fibers (pulp fibers), and the pulverized fibers consist only of hardwood fibers (100% hardwood fibers). Note that fibers included in the absorbent core 11 are not limited to only the pulverized fibers consisting of the hardwood fibers, and the pulverized fibers may include, in addition to the hardwood fibers, pulp fibers such as softwood fibers made from softwood. The absorbent core 11 may also include liquid-absorbent fibers, such as cellulose-based absorbent fibers, and synthetic fibers such as polyolefin, polyester, acrylic, and acrylate, as well as superabsorbent polymers (SAP). FIG. 4A is a plan view of the absorbent core 11. FIG. 4B is a schematic cross-sectional view taken along J-J in FIG. 4A. FIG. 5 is an enlarged cross-sectional schematic view taken along line K-K in FIG. 4A. FIG. 6 is an enlarged view of the absorbent body 10 in FIG. 3. In FIGS. 4A, 4B, and FIG. 5, unevenness caused by compressed portions such as compressed portions EL, is omitted.

The absorbent core 11 includes a middle-high portion 11d in which the basis weight of the liquid-absorbent fibers is higher than that in the surrounding region. The middle-high portion 11d is a portion in which the thickness of the absorbent core 11 is greater (the length in the thickness direction is longer) than that of the surrounding region. The middle-high portion 11d of the present embodiment is a portion that protrudes toward the skin side in the thickness direction and has a substantially elliptical shape in a plan view. The middle-high portion 11d is provided, in the central portion of the

absorbent core 11 in the width direction, at a position overlapping the crotch region CA in a plan view, thereby making the middle-high portion 11d likely to come into contact with the excretion opening of the wearer in the put-on state. This allows the sanitary napkin 1 to fit more readily the wearer’s body while facilitating quick absorption of excrement. Furthermore, since the middle-high portion 11d with a high basis weight of the liquid-absorbent fibers is provided at a position overlapping the crotch region CA in a plan view, the excrement discharged from the excretion opening of the wearer is likely to be retained inside the absorbent core 11, in the put-on state.

The core-wrapping sheet 12 is a liquid-permeable sheet member. The core-wrapping sheet 12 of the present embodiment is tissue containing cellulose fibers. The core-wrapping sheet 12 covers the absorbent core 11, thereby maintaining the shape of the absorbent core 11 and promoting the diffusion of liquid into the absorbent core 11.

As shown in FIG. 6, the core-wrapping sheet 12 includes: a skin-side portion 12a located on the skin side with respect to the absorbent core 11; and a non-skin-side portion 12b located on the non-skin side with respect to the absorbent core 11. In the present embodiment, as shown in FIG. 6 and the like, both end portions of the core-wrapping sheet 12 are folded back to the skin side of the absorbent core 11, thereby allowing the core-wrapping sheet 12 to cover both the skin-side surface and the non-skin-side surface of the absorbent core 11. Specifically, the absorbent core 11 is wrapped with the core-wrapping sheet 12 so as to cover both side ends of the absorbent core 11 in the width direction, with a leading end 12ea extending in the longitudinal direction positioned on the outer side and a leading end 12eb extending in the longitudinal direction positioned on the inner side. However, the shape and form of the core-wrapping sheet 12 are not limited thereto. For example, the core-wrapping sheet 12 may be configured such that two sheet members are arranged on the skin side and the non-skin side of the absorbent core 11, respectively. In the present embodiment, an adhesive is not provided on the inward side of the core-wrapping sheet 12. In other words, the absorbent core 11 is not fixed to the core-wrapping sheet 12 with an adhesive, and the shape of the absorbent core 11 is maintained by being wrapped with the core-wrapping sheet 12.

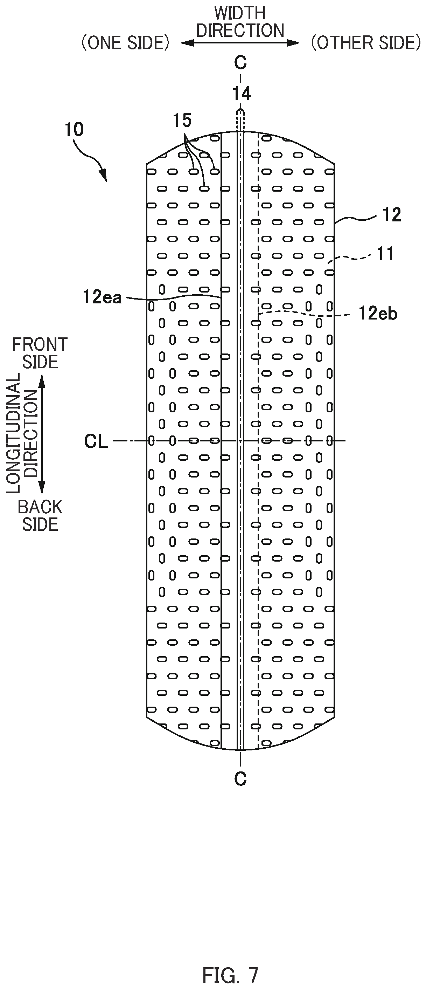

FIG. 7 is a plan view of the absorbent body 10 as viewed from the skin side. In FIG. 7, the middle-high portion 11d provided in the absorbent core 11 is omitted. As shown in FIG. 7, the absorbent body 10 includes a central compressed portion 14 and a plurality of absorbent-body compressed portions 15. Each of the central compressed portion 14 and the absorbent-body compressed portion 15 is a portion in which the absorbent core 11 and the core-wrapping sheet 12 are compressed in the thickness direction.

The central compressed portion 14 and the absorbent-body compressed portion 15 are formed, in the manufacturing process, by passing the absorbent body 10 through a roll gap between a protrusion roller having a plurality of protrusion portions and an anvil roller having a flat surface, and compressing this absorbent body 10. The central compressed portion 14 and the absorbent-body compressed portions 15 of the present embodiment are portions in which the absorbent body 10 has been pressed from the non-skin side toward the skin side by passing the absorbent body 10 through the roll gap in the state where the protrusion roller comes into contact with the non-skin-side surface of the absorbent body 10 and the anvil roller comes into contact with the skin-side surface of the absorbent body 10.

The central compressed portion 14 is a line-like compressed portion that is provided in the widthwise central portion and extends in the longitudinal direction. The central compressed portion 14 is a portion that extends from the front end to the back end of the absorbent body 10 and is recessed in the thickness direction from the non-skin side or the skin side. The central compressed portion 14 serves as a guide portion that guides the absorbent body 10 so as to bend toward the skin side in the thickness direction at the central compressed portion 14. Since the central compressed portion 14 is a portion having a higher fiber density than non-compressed portions, the central compressed portion 14 also serves as a guide portion that guides excrement in the longitudinal direction by capillary action.

The absorbent-body compressed portions 15 are compressed portions arranged in a substantially staggered pattern over the entire region of the absorbent body 10. In the present embodiment, the absorbent-body compressed portions 15 include a plurality of compressed portions having an elliptical shape. As with the central compressed portion 14, the absorbent-body compressed portion 15 is also a portion recessed in the thickness direction from the non-skin side or the skin side. By providing the absorbent-body compressed portions 15, it becomes easier to maintain the shape of the absorbent body 10. Furthermore, the absorbent-body compressed portions 15, having a higher fiber density than non-compressed portions, function as portions of the absorbent core 11 that are more likely to absorb excrement by capillary action. Due to a density difference between the absorbent-body compressed portions 15 and the non-compressed portions, it becomes easier to suppress diffusion of an oil agent Y in the absorbent core 11, as will be described later.

As shown in FIG. 1 and the like, the sanitary napkin 1 includes line-like compressed portions EL and circular compressed portions ee.

Each of the line-like compressed portions EL is a portion in which at least a part of the top sheet 21, the second sheet 22, and the absorbent body 10 is compressed in the thickness direction. In the present embodiment, the line-like compressed portions EL of the sanitary napkin 1 include: a front compressed portion ELa provided on the front side with respect to a longitudinal center line CL; a back compressed portion ELb provided on the back side with respect to the center line CL; and a central compressed portion ELc provided between the front compressed portion ELa and the back compressed portion ELb. By providing the line-like compressed portions EL, the joining of members in the thickness

direction can be strengthened, thereby increasing the fiber density to improve liquid absorbency. The line-like compressed portions EL are line-like compressed portions extending in the longitudinal direction in a plan view, and are provided as a pair with a predetermined spacing in the width direction. A plurality of circular compressed portions ee are provided in the widthwise central portion and at a position overlapping the crotch region CA in the longitudinal direction. The circular compressed portions ee are compressed portions having a circular shape in a plan view, and are portions in which at least a part of the top sheet 21, the second sheet 22, and the absorbent body 10 is compressed in the thickness direction.

The line-like compressed portion EL and the circular compressed portion ee are portions in which members stacked in the thickness direction are pressed and squashed. Therefore, the fiber density of each of the members in each compressed portion is higher than the fiber density in a region in which the compressed portion is not provided. The line-like compressed portion EL and the circular compressed portion ee are formed, in the manufacturing process, by passing the sanitary napkin 1 through the roll gap between a protrusion roller having a plurality of protrusion portions and an anvil roller having a flat surface, and compressing this sanitary napkin 1.

Diffusion of Excrement in Sanitary Napkin 1

When an absorbent article such as the sanitary napkin 1 is put on, it is preferable that the absorbent article quickly absorb excrement such as menstrual blood discharged from the wearer's vaginal opening and diffuse the absorbed excrement over a wide range in the absorbent article, thereby making the excrement less likely to remain on the skin-side surface of the absorbent article to reduce adverse effects and discomfort on the wearer's skin. In particular, sanitary napkins are required to have the ability to absorb and diffuse excrement in order to absorb the excrement such as highly viscous menstrual blood. Therefore, in the absorbent article, means for providing a substance or the like that promotes diffusion of excrement has conventionally been known. However, there has been a concern that using a substance or the like that promotes diffusion of excrement may cause the excrement to diffuse excessively within the absorbent article, thereby causing the excrement to leak from the absorbent article.

In the sanitary napkin 1 of the present invention, the oil agent Y that promotes diffusion of excrement is provided in the absorbent core 11. Furthermore, the absorbent core 11 of the sanitary napkin 1 has pulverized fibers that include hardwood fibers made from hardwood. In either the longitudinal direction or the width direction, when the absorbent core 11 is divided into three equal regions, these regions are, in order from one side, a one-side portion, a central portion, and an other-side portion. The basis weight of the oil agent Y provided in the central portion is higher than the basis weight of the oil agent Y provided in the one-side portion.

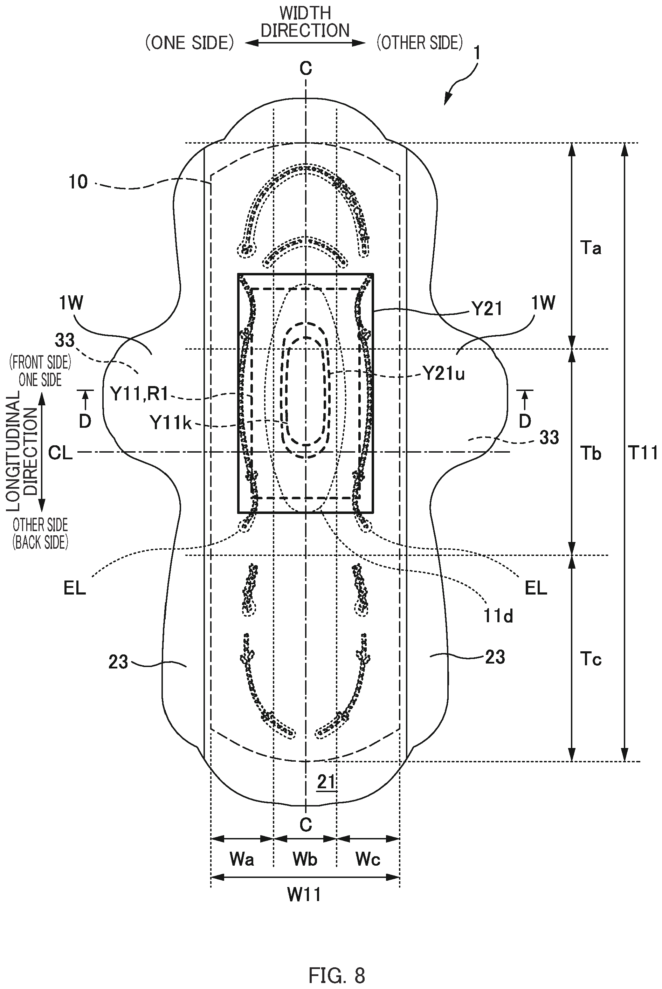

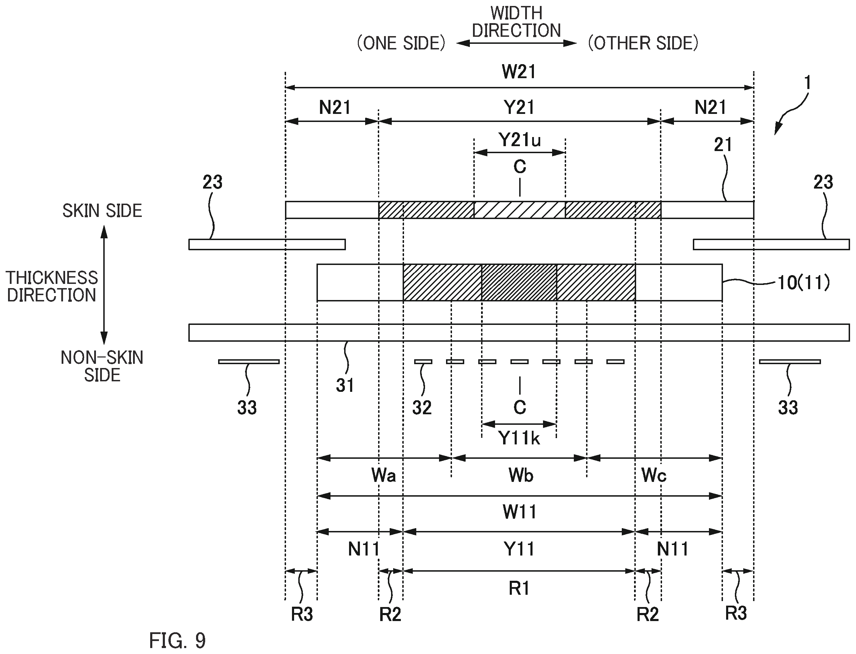

In the sanitary napkin 1 of the present embodiment, the oil agent Y is applied to a region Y11 from the skin-side surface of the top sheet 21. FIG. 8 is a schematic plan view illustrating the region in which the oil agent Y is provided. FIG. 9 is a schematic cross-sectional view illustrating the region in which the oil agent Y is provided. FIGS. 8 and 9 are schematic views, in which, for convenience, the members are omitted and/or simplified. FIG. 8 and the like illustrate regions of the sanitary napkin 1 in which the oil agent Y is provided. The region Y11 refers to a region of the absorbent core 11 in which the oil agent Y is provided (hereinafter referred to as a "core-oil-agent-present region Y11"), and a region Y21 refers to a region of the top sheet 21 in which the oil agent Y is provided (hereinafter referred to as a "surface-oil-agent-present region Y21"). In FIG. 9, the regions in which the oil agent Y is provided are indicated by downward-left hatched portions. However, FIG. 9 merely illustrates a planar arrangement of the oil agent Y, and the oil agent Y is not necessarily provided over the entire region of each member in the thickness direction. For example, it is acceptable that the oil agent Y is provided only in the skin-side portion of the absorbent core 11, while the oil agent Y is not provided in the non-skin-side portion of the absorbent core 11.

As shown in FIG. 8, in the longitudinal direction of the sanitary napkin 1, when the absorbent core 11 is divided into three equal portions, these portions are, in order from the front side (one side), a front-side portion (one-side portion) Ta, a central portion Tb, and a back-side portion (other-side portion) Tc. The absorbent article has a feature that the basis weight of the oil agent Y (i.e., the weight of the oil agent Y per unit area) provided in the central portion Tb is higher than the basis weight of the oil agent provided in the front-side portion Ta. Alternatively, in the sanitary napkin 1 in which the absorbent core 11 includes the hardwood fibers and the oil agent Y, as shown in FIG. 8, in the width direction of the sanitary napkin 1, when the absorbent core 11 is divided into three equal portions, these portions are, in order from one side, a left-side portion (one-side portion) Wa, a central portion Wb, and a right-side portion (other-side portion) Wc. In addition, the absorbent article has a feature in that the basis weight of the oil agent Y provided in the central portion Wb is higher than the basis weight of the oil agent provided in the left-side portion Wa. The left-side portion Wa of the sanitary napkin 1 corresponds to, in FIG. 8, the left side of the wearer during wear.

In FIG. 8, in the longitudinal direction, the front-side portion Ta is referred to as the one-side portion and the back-side portion Tc is referred to as the other-side portion; however, the configuration is not limited thereto. The front-side portion Ta may be referred to as the other-side portion, and the back-side portion Tc may be referred to as the one-side portion. Similarly, in the width direction, the left-side portion Wa is referred to as the one-side portion and the right-side portion Wc is referred to as the other-side portion; however, the configuration is not limited thereto. The left-side portion Wa

may be referred to as the other-side portion and the right-side portion Wc may be referred to as the one-side portion.

In the present embodiment, in the absorbent core 11 of the sanitary napkin 1, the region Y11 in which the oil agent Y is provided is located in a substantially central portion of the sanitary napkin 1.

Specifically, in the longitudinal direction, most of the region Y11 is located in the longitudinal central portion Tb, and a part of the oil agent Y is provided in the longitudinal front-side portion Ta. The oil agent Y is not provided in the longitudinal back-side portion Tc. In the longitudinal direction, the basis weight of the oil agent Y provided in the central portion Tb (the weight of the oil agent Y in the central portion Tb divided by the area of the central portion Tb) is greater than the basis weight of the oil agent Y provided in the front-side portion Ta (the weight of the oil agent Y in the front-side portion Ta divided by the area of the front-side portion Ta). In the longitudinal direction, the basis weight of the oil agent Y provided in the central portion Tb (the weight of the oil agent Y in the central portion Tb divided by the area of the central portion Tb) is greater than the basis weight of the oil agent Y provided in the back-side portion Tc (the weight of the oil agent Y in the back-side portion Tc divided by the area of the back-side portion Tc). In the width direction, most of the region Y11 is located in the widthwise central portion Wb, a part of the oil agent Y is provided in the widthwise left-side portion Wa, and a part of the oil agent Y is provided in the widthwise right-side portion Wc. In the width direction, the basis weight of the oil agent Y provided in the central portion Wb (the weight of the oil agent Y in the central portion Wb divided by the area of the central portion Wb) is greater than the basis weight of the oil agent Y provided in the left-side portion Wa (the weight of the oil agent Y in the left-side portion Wa divided by the area of the left-side portion Wa). In the width direction, the basis weight of the oil agent Y provided in the central portion Wb (the weight of the oil agent Y in the central portion Wb divided by the area of the central portion Wb) is greater than the basis weight of the oil agent Y provided in the right-side portion Wc (the weight of the oil agent Y in the right-side portion Wc divided by the area of the right-side portion Wc).

In the sanitary napkin 1, in either the longitudinal direction or the width direction of the absorbent core 11, the basis weight of the oil agent Y in the central portion is higher than the basis weight of the oil agent Y in the one-side portion, thereby promoting diffusion of excrement in the central portion more readily than in the one-side portion of the absorbent core 11. On the other hand, in either the longitudinal direction or the width direction, the basis weight of the oil agent Y in the one-side portion is lower than the basis weight of the oil agent Y in the central portion, thereby suppressing diffusion of excrement in the one-side portion more readily than in the central portion of the absorbent core 11. In general, fibers made from hardwood have a shorter fiber length than softwood fibers. In the absorbent core 11, the longer the fiber length, the more readily the diffusion is promoted. Thus, by including hardwood fibers as pulverized fibers in the absorbent core 11, diffusion of excrement in the one-side portion, where the basis weight of oil agent Y is low, can be more readily suppressed. Even in the case where the absorbent core 11 includes hardwood fibers as the pulverized fibers, by making, in either the longitudinal direction or the width direction, the basis weight of the oil agent in the central portion higher than that of the oil agent Y in the one-side portion, diffusion of excrement in the central portion of the absorbent core 11 is readily promoted. This can reduce the risk that excrement remains on the surface of the absorbent article during wear. Accordingly, in the sanitary napkin 1, by promoting the diffusion of excrement in the central portion of the absorbent core 11, excrement is less likely to remain on the skin-side surface of the absorbent core 11, thereby reducing adverse effects and discomfort to the wearer's skin and reducing the risk that excrement leaks outward from the one-side end of the sanitary napkin 1 in the longitudinal or the width direction. In addition, during wear, the wearer visually recognizes the state in which excrement is more likely to diffuse in the central portion of the absorbent core 11 and less likely to diffuse in the one-side portion in the longitudinal direction or the width direction, thereby making the wearer's concern that the excrement may leak from the sanitary napkin 1 likely to be alleviated.

Examples of the pulverized fibers include: pulp such as wood pulp obtained from softwood (e.g., Southern yellow pine) or hardwood (e.g., Eucalyptus), and non-wood pulp such as bagasse, kenaf, bamboo, hemp, and cotton (e.g., cotton linter); a regenerated cellulose fiber such as a rayon fiber; and a semi-synthetic fiber such as an acetate fiber. In the absorbent article, softwood fibers (softwood pulp), which have a relatively long fiber length, are often used.

The average fiber length of the softwood fibers (softwood pulp) is 2.5 mm, and the average fiber length of the hardwood fibers (hardwood pulp) is 0.79 mm. In other words, the average fiber length of the hardwood fibers is shorter than the average fiber length of the softwood fibers. The hardwood fibers used as the pulverized fibers in the absorbent core 11 are less likely to promote diffusion of excrement (liquid) compared to the softwood fibers. Thus, by providing the oil agent Y in the central portion, in the longitudinal direction or the width direction, of the absorbent core 11 having the hardwood fibers, the diffusion is promoted while suppressing the diffusion of excrement more readily in the one-side portion in the longitudinal direction or the width direction where the basis weight of the oil agent Y is low. As a result, leakage of excrement can be reduced.

In the present embodiment, the pulverized fibers of the absorbent core 11 are formed solely from hardwood fibers (hardwood pulp). That is, all of the pulverized fibers in the absorbent core 11 are hardwood fibers (100% hardwood fibers). As a result, the oil agent Y promotes diffusion of excrement in the central portion of the absorbent core 11 in the longitudinal direction or the width direction, whereas diffusion of excrement is more readily suppressed in the one-side portion in the longitudinal direction or the width direction, which is a region in which the basis weight of the oil agent Y is low and which has hardwood fibers with a short average fiber length.

The pulverized fibers of the absorbent core 11 may include, in addition to hardwood pulp, softwood fibers (softwood pulp) formed from softwood. In the case where the absorbent core 11 has softwood fibers, it is preferable that the total weight of the hardwood fibers is greater than the total weight of the softwood fibers. The absorbent core 11 has a large amount of hardwood fibers with a short average fiber length, thereby making it easier, due to the oil agent Y, to promote absorption of excrement in the central portion of the absorbent core 11 while making it easier to suppress diffusion of excrement in the one-side portion of the absorbent core 11 in the longitudinal direction or the width direction.

The presence of hardwood fibers in the pulverized fibers of the absorbent core 11 can be confirmed by known methods. For example, the following method may be used.

First, a predetermined weight (0.04 g) of pulverized fibers of the absorbent core 11 of the sanitary napkin 1 is taken out as a sample, and the pulverized fibers are disintegrated with water (distilled water).

Next, the sample is transferred to a beaker, and using a fiber length distribution analyzer ("Valmet FS5", manufactured by Valmet Corporation), an image is captured and a fiber length is measured. In the device, the concentration of the sample is automatically adjusted to 15 to 23 mg/L. The presence of hardwood fibers in the sample can be confirmed by comparing the obtained fiber length with an average fiber length of hardwood fibers.

It is preferable that 50% or more of the pulverized fibers in the absorbent core 11, on a length-weighted average basis, have the fiber length of 1 mm or less. The absorbent core 11 includes a large amount of fibers with a short fiber length, thereby reducing planar diffusion of excrement along the fibers in the absorbent core 11. As a result, it becomes easier to promote transfer of excrement from the skin side to the non-skin side of the absorbent core 11, thereby making it easier to reduce the risk that excrement leaks to the outside of the sanitary napkin 1.

The oil agent Y is a substance that promotes diffusion of excrement (liquid), and it is preferable that the oil agent Y be an amphiphilic substance. Since the oil agent Y is an amphiphilic substance having both hydrophilic and hydrophobic groups, a contact between the oil agent Y and excrement during wear changes the surface tension of the excrement, thereby making it easier to promote diffusion of excrement (liquid) in the region to which the oil agent Y is applied.

As the oil agent Y, for example, the following can be used.

[Ester of chain hydrocarbon tetraol and at least one fatty acid]

- UNISTER H-408BRS, manufactured by NOF CORPORATION Pentaerythritol tetra(2-ethylhexanoate), weight-average molecular weight: approximately 640
- UNISTER H-2408BRS-22, manufactured by NOF CORPORATION Mixture of pentaerythritol tetra(2-ethylhexanoate) and neopentylglycol di(2-ethylhexanoate) (58:42, weight ratio), weight-average molecular weight: approximately 520

[Ester of chain hydrocarbon triol and at least one fatty acid]

- Tri-C2L oil fatty acid glyceride, manufactured by NOF CORPORATION

Triester of glycerin and fatty acids, C8 fatty acid:C10 fatty acid:C12 fatty acid at a weight ratio of about 37:7:56, weight-average molecular weight: approximately 570

- Tri-CL oil fatty acid glyceride, manufactured by NOF CORPORATION

Triester of glycerin and fatty acids, C8 fatty acid:C12 fatty acid at a weight ratio of about 44:56, weight-average molecular weight: approximately 570

- PANACET 810s, manufactured by NOF CORPORATION

Triester of glycerin and fatty acids, C8 fatty acid:C10 fatty acid at a weight ratio of about 85:15, weight-average molecular weight: approximately 480

- PANACET 800, manufactured by NOF CORPORATION

Triester of glycerin and fatty acids, all of the fatty acids being octanoic acid (C8), weight-average molecular weight: approximately 470

- PANACET 800B, manufactured by NOF CORPORATION

Triester of glycerin and fatty acids, all of the fatty acids being 2-ethylhexanoic acid (C8), weight-average molecular weight: approximately 470

- NA36, manufactured by NOF CORPORATION

Triester of glycerin and fatty acids, C16 fatty acid:C18 fatty acid:C20 fatty acid (including both saturated fatty acids and unsaturated fatty acids) at a weight ratio of about 5:92:3, weight-average molecular weight: approximately 880

- Tri-coconut fatty acid glyceride, manufactured by NOF CORPORATION

  Triester of glycerin and fatty acids, C8 fatty acid:C10 fatty acid:C12 fatty acid:C14 fatty acid:C16 fatty acid (including both saturated fatty acids and unsaturated fatty acids) at a weight ratio of about 4:8:60:25:3, weight-average molecular weight: approximately 670
  Caprylic acid diglyceride, manufactured by NOF CORPORATION Glycerin diester with octanoic acid as the fatty acid, weight-average molecular weight: approximately 340

[Ester of chain hydrocarbon diol and at least one fatty acid]

- UNISTER H-208BRS, manufactured by NOF CORPORATION Neopentylglycol di(2-ethylhexanoate), weight-average molecular weight: approximately 360
- COMPOL BL, manufactured by NOF CORPORATION

  Butylene glycol monoester of dodecanoic acid (C12), weight-average molecular weight: approximately 270
  COMPOL BS, manufactured by NOF CORPORATION
  Butylene glycol monoester of octadecanoic acid (C18), weight-average molecular weight: approximately 350

[Compound having a chain hydrocarbon moiety and two to four hydroxyl groups substituting hydrogen atoms on the chain hydrocarbon moiety]

[Ether of a compound having a chain hydrocarbon moiety and one hydroxyl group substituting a hydrogen atom on the chain hydrocarbon moiety]

[Carboxylic acid, hydroxy acid, alkoxy acid or oxoacid having a chain hydrocarbon moiety and two to four carboxyl groups substituting hydrogen atoms on the chain hydrocarbon moiety]

[Ester of chain hydrocarbon tricarboxylic acid, hydroxy acid, alkoxy acid or oxoacid having three carboxyl groups, and at least one aliphatic monohydric alcohol]

- Tributyl O-acetylcitrate, manufactured by Tokyo Chemical Industry Co., Ltd.

Weight-average molecular weight: approximately 400

- Tributyl citrate, manufactured by Tokyo Chemical Industry Co., Ltd.

Weight-average molecular weight: approximately 360

[Ester of chain hydrocarbon dicarboxylic acid, hydroxy acid, alkoxy acid or oxoacid having two carboxyl groups, and at least one aliphatic monohydric alcohol]

- Dioctyl adipate, manufactured by Wako Pure Chemical Corporation

Weight-average molecular weight: approximately 380

[Compound having a chain hydrocarbon moiety and one bond selected from the group consisting of an ether bond (-O-), carbonyl bond (-CO-), ester bond (-COO-) and carbonate bond (-OCOO-), inserted between a C-C single bond of the chain hydrocarbon moiety]

[Ester of a fatty acid and an aliphatic monohydric alcohol]

- ELECTOL WE20, manufactured by NOF CORPORATION

Ester of dodecanoic acid (C12) and dodecyl alcohol (C12), weight-average molecular weight: approximately 360

- ELECTOL WE40, manufactured by NOF CORPORATION

Ester of tetradecanoic acid (C14) and dodecyl alcohol (C12), weight-average molecular weight: approximately 390

[Polyoxy C3-C6 alkylene glycol, or alkyl ester or alkyl ether thereof]

[Polyoxy C3-C6 alkylene glycol]

- UNIOL PB500, manufactured by NOF CORPORATION Polybutylene glycol, weight-average molecular weight: approximately 500
- UNIOL PB700, manufactured by NOF CORPORATION Polyoxybutylenepolyoxypropylene glycol, weight-average molecular weight: approximately 700

[Chain hydrocarbon]

- PARLEAM 6, manufactured by NOF CORPORATION

Branched hydrocarbon produced by copolymerization of liquid isoparaffin, isobutene, and n-butene, followed by hydrogen addition, having a polymerization degree: approximately 5 to 10, weight-average molecular weight: approximately 330

Others

- NA50, manufactured by NOF CORPORATION

Triester of glycerin and fatty acids obtained by addition of hydrogen to NA36, thereby reducing the proportion of double bonds derived from unsaturated fatty acid starting material, weight-average molecular weight: approximately 880

- (Caprylic acid/capric acid) monoglyceride, manufactured by NOF CORPORATION

Monoester of glycerin and fatty acids, with octanoic acid (C8) and decanoic acid (C10) at a weight ratio of about 85:15, weight-average molecular weight: approximately 220

- Monomuls 90-L2, lauric acid monoglyceride, manufactured by Cognis Japan Ltd.
- Isopropyl citrate, manufactured by Tokyo Chemical Industry Co., Ltd.

Weight-average molecular weight: approximately 230

- Diisostearyl malate

Weight-average molecular weight: approximately 640

- UNIOL PB1000R, manufactured by NOF CORPORATION Polybutylene glycol, weight-average molecular weight: approximately 1000
- UNIOL D-250, manufactured by NOF CORPORATION Polypropylene glycol, weight-average molecular weight: approximately 250
- UNIOL D-400, manufactured by NOF CORPORATION Polypropylene glycol, weight-average molecular weight: approximately 400
- UNIOL D-700, manufactured by NOF CORPORATION Polypropylene glycol, weight-average molecular weight: approximately 700
- UNIOL D-1000, manufactured by NOF CORPORATION Polypropylene glycol, weight-average molecular weight: approximately 1,000
- UNIOL D-1200, manufactured by NOF CORPORATION Polypropylene glycol, weight-average molecular weight: approximately 1,160
- UNIOL D-2000, manufactured by NOF CORPORATION Polypropylene glycol, weight-average molecular weight: approximately 2,030
- UNIOL D-3000, manufactured by NOF CORPORATION Polypropylene glycol, weight-average molecular weight: approximately 3,000
- UNIOL D-4000, manufactured by NOF CORPORATION Polypropylene glycol, weight-average molecular weight: approximately 4,000

•PEG1500, manufactured by NOF CORPORATION

Polyethylene glycol, weight-average molecular weight: approximately 1,500 to 1,600

- WILBRITE cp9, manufactured by NOF CORPORATION Polybutylene glycol compound whose OH groups at both ends are esterified with hexadecanoic acid (C16), weight-average molecular weight: approximately 1,150
- UNILUBE MS-70K, manufactured by NOF CORPORATION Stearyl ether of polypropylene glycol having approximately 15 repeating units, weight-average molecular weight: approximately 1,140
- NONION S-6, manufactured by NOF CORPORATION Polyoxyethylene monostearate, approximately 7 repeating units, weight average-molecular weight: approximately 880
- UNILUBE 5TP-300KB Polyoxyethylenepolyoxypropylene pentaerythritol ether produced by addition of 5 mol of ethylene oxide and 65 mol of propylene oxide to 1 mol of pentaerythritol, weight-average molecular weight: 4,130

WILBRITE s753, manufactured by NOF CORPORATION Polyoxyethylene polyoxypropylene polyoxybutylene glycerin, weight-average molecular weight: approximately 960

- UNIOL TG-330, manufactured by NOF CORPORATION Glyceryl ether of polypropylene glycol, approximately 6 repeating units, weight-average molecular weight: approximately 330
- UNIOL TG-1000, manufactured by NOF CORPORATION Glyceryl ether of polypropylene glycol, approximately 16 repeating units, weight-average molecular weight: approximately 1,000
- UNIOL TG-3000, manufactured by NOF CORPORATION Glyceryl ether of polypropylene glycol, approximately 16 repeating units, weight-average molecular weight: approximately 3,000
- UNIOL TG-4000, manufactured by NOF CORPORATION Glyceryl ether of polypropylene glycol, approximately 16 repeating units, weight-average molecular weight: approximately 4,000
- UNILUBE DGP-700, manufactured by NOF CORPORATION Diglyceryl ether of polypropylene glycol, approximately 9 repeating units, weight-average molecular weight: approximately 700
- UNIOX HC60, manufactured by NOF CORPORATION Polyoxyethylene hydrogenated castor oil, weight-average molecular weight: approximately 3,570
- Vaseline, manufactured by Cognis Japan Ltd. Petroleum-derived hydrocarbon, semi-solid

The comparison of the basis weight of the oil agent Y can be performed using a known method. For example, a reagent corresponding to the oil agent Y may be applied, and comparison may be made based on color change or the light/dark tone difference.

The sanitary napkin 1 of the present embodiment uses, as the oil agent Y, PANACET 810S (triglyceride, manufactured by NOF CORPORATION). The oil agent Y is an oil agent that contains unsaturated fatty acids, and this oil

agent can promote diffusion of excrement, improve the feel by imparting unsaturated fatty acids to the wearer's skin, and protect the wearer's skin.

Using an oil leakage color-developing inspection agent, "Moraymille Oil (manufactured by TASETO Co., Ltd.)", it is possible to compare the presence of the oil agent Y and the basis weight thereof.

Specifically, the absorbent core 11 is taken out from the sanitary napkin 1, and the aforementioned oil leakage color-developing inspection agent is sprayed over the entire region of the absorbent core 11. When the oil leakage color-developing inspection agent is applied, a region in which the oil (oil agent Y) is present develops a red color. The regions that do not develop a red color indicate that the oil agent Y is not applied, and that the basis weight of the oil agent Y is zero. On the other hand, in regions that develop a red color, the basis weight of the oil agent Y in each portion can be compared by comparing the lightness/darkness of the red color.

In the sanitary napkin 1 of the present embodiment, in the longitudinal direction and the width direction, the basis weight of the oil agent Y provided in a central portion is higher than the basis weight of the oil agent Y provided in a one-side portion. In other words, the basis weight of the oil agent Y provided in the central portion Tb is higher than the basis weight of the oil agent Y provided in the front-side portion Ta, and the basis weight of the oil agent Y provided in the central portion Wb is higher than the basis weight of the oil agent provided in the left-side portion Wa. As a result, the central portion (corresponding to the central portion Tb and the central portion Wb) of the absorbent core 11 in the longitudinal direction and the width direction is a portion that is likely to come into contact with the excretion opening such as the vaginal opening of the wearer, during wear. Therefore, a high basis weight of the oil agent Y in the central portion of the absorbent core 11 in the longitudinal direction and the width direction can facilitate quick absorption of excrement discharged during wear and diffusion of the excrement. On the other hand, by making it easier to suppress diffusion of the excrement in the one-side portions Ta and Wa of the absorbent core 11 in the longitudinal direction and the width direction, it is possible to reduce a risk that excrement leaks from the front outer side (one side) of the sanitary napkin 1 in the longitudinal direction, and from the left outer side (one side) of the sanitary napkin 1 in the width direction.

Furthermore, it is preferable that the oil agent Y is not provided in the outer end portion of the front-side portion (one-side portion) Ta of the absorbent core 11 in the longitudinal direction, and the outer end portion of the left-side portion (one-side portion) Wa of the absorbent core 11 in the width direction. As a result, it becomes easier to suppress the diffusion of excrement in the outer end portion of the front-side portion (one-side portion) Ta of the absorbent core 11 in the longitudinal direction, and in the outer end portion of the left-side portion (one-side portion) Wa of the absorbent core 11 in the width direction. This can reduce the risk that excrement leaks from the front outer side (one side) of the sanitary napkin 1 in the longitudinal direction and from the left outer side (one side) of the sanitary napkin 1 in the width direction. The outer end portion of the front-side portion (one-side portion) Ta of the absorbent core 11 in the longitudinal direction refers to a portion having a length of 10 mm from the longitudinal front end of the absorbent core 11, and more preferably refers to a portion having a length of 5 mm from the longitudinal front end. The outer end portion of the left-side portion (one-side portion) Wa of the absorbent core 11 in the width direction refers to a portion having a length of 10 mm from the widthwise left end of the absorbent core 11, and more preferably refers to a portion having a length of 5 mm from the widthwise left end.

It is also preferable that the sanitary napkin 1 has line-like compressed portions (compressed portions) EL in which the absorbent core 11 is compressed in the thickness direction.

As described above, the line-like compressed portions EL of the sanitary napkin 1 include back compressed portions ELb located on the back side with respect to the center line CL in the longitudinal direction (FIG. 1). In such a case, it is preferable that the back end of the region (Y11) in which the oil agent Y is provided, is located on the front side with respect to the front end of the back compressed portion ELb. The line-like compressed portions EL and the back compressed portions ELb are portions having a higher fiber density than the surrounding region, and are portions that tend to promote diffusion of excrement due to capillary action. Thus, by providing the oil agent Y on the front side with respect to the front end of the back compressed portion ELb, it becomes easier to suppress the diffusion on the back side of the absorbent core 11, thereby reducing the risk that excrement leaks outward on the back side of the sanitary napkin 1.

As described above, the sanitary napkin 1 includes the core-wrapping sheet 12 that covers the absorbent core 11. In the core-wrapping sheet 12, it is preferable that the average fiber length of fibers of the skin-side portion 12a (the core-wrapping sheet), which is located on the skin side with respect to the absorbent core 11, be equal to or greater than the average fiber length of the pulverized fibers of the absorbent core 11. As a result, compared to the case where the average fiber length of fibers of the skin-side portion 12a is shorter than the average fiber length of the pulverized fibers of the absorbent core 11, it becomes easier to promote absorption and diffusion of excrement in the skin-side portion 12a during wear. Therefore, by diffusing the excrement in a planar manner in the skin-side portion 12a and then vertically transferring the excrement to the absorbent core 11 located on the non-skin side of the skin-side portion 12a, penetration of the excrement into the absorbent core 11 can be more readily promoted. The average fiber length of the pulverized fibers of the absorbent core 11 and the average fiber length of the fibers of the skin-side portion 12a can be measured by a known method, and, for example, can be obtained by capturing images using the above-described fiber length distribution analyzer ("Valmet FS5", manufactured by Valmet Corporation) and measuring the fiber length.

The fibers of the skin-side portion 12a (core-wrapping sheet) can be extracted, for example, by the following method.

First, the sanitary napkin 1 is disintegrated with toluene to obtain the core-wrapping sheet 12.

The extracted core-wrapping sheet is dried.

Then, 15 g of the core-wrapping sheet and approximately 1.5 L of water, with the water adjusted so that the concentration is 1% relative to the weight of the core-wrapping sheet, are placed in a plastic container (inner diameter: 120 mm, height: 220 mm) and stirred at room temperature for about 24 hours using a stirrer (EYELA MAZELA Z, manufactured by TOKYO RIKAKIKAI CO., LTD.). At this time, the fibers are stirred slowly such that the fibers are not broken.

After that, the fibers dispersed in the water are aspirated together with the water using a dropper (hole diameter: 2 to 3 mm).

The extracted fibers are collected by suction filtration and then dried.

Using an electron microscope, the fiber length of the extracted fibers is measured, and the average fiber length is calculated from the measured fiber lengths.

In the skin-side portion (core-wrapping sheet) 12a of the core-wrapping sheet 12, which is located on the skin side with respect to the absorbent core 11 (see FIG. 6), it is preferable that an adhesive is not provided between the skin-side surface of the absorbent core 11 and the non-skin-side surface of the skin-side portion 12a. In other words, it is preferable that the skin-side surface of the absorbent core 11 and the skin-side portion 12a are not joined with an adhesive. The oil agent Y that promotes diffusion of excrement is also likely to be compatible with not only the excrement but also the adhesive. In the case where the adhesive is provided between the skin-side surface of the absorbent core 11 and the non-skin-side surface of the skin-side portion 12a, then, before the sanitary napkin 1 is put on (i.e., before excrement is absorbed), the oil agent Y in the skin-side portion 12a is compatible with the adhesive, thereby causing the oil agent Y to diffuse into an unintended region of the skin-side portion 12a. In this case, during wear of the sanitary napkin 1, when the excrement is absorbed, the excrement that has diffused in a planar manner in the skin-side portion 12a moves toward the non-skin side into the absorbent core 11, thereby causing the excrement to diffuse excessively over a wide region within the absorbent core 11. This may result in leakage of the excrement from the sanitary napkin 1. In contrast, an adhesive is not provided between the skin-side surface of the absorbent core 11 and the non-skin-side surface of the skin-side portion 12a, thereby preventing diffusion of the oil agent Y due to its compatibility with the adhesive in the skin-side portion 12a. This can reduce the risk of excessive diffusion of excrement in the absorbent core 11 during wear, and can consequently reduce the risk that excrement leaks to the outside of the sanitary napkin 1.

For the absorbent core 11 and the core-wrapping sheet 12, it is preferable that the adhesive is not provided between the non-skin-side surface of the absorbent core 11 and the non-skin-side portion (core-wrapping sheet) 12b located on the non-skin side with respect to the absorbent core 11. In other words, it is preferable that the non-skin-side surface of the absorbent core 11 and the non-skin-side portion 12b are not adhered with an adhesive. On the non-skin-side surface of the absorbent core 11 as well, similarly to the skin-side surface (skin-side portion 12a), it is possible to prevent excessive diffusion of the oil agent Y in the non-skin-side portion 12b due to compatibility of the oil agent Y with the adhesive, thereby reducing the risk that excrement that has diffused in a planar manner in the non-skin-side portion 12b moves into the absorbent core 11 and diffuses excessively over a wide region within the absorbent core 11. This can reduce the risk that excrement leaks to the outside of the sanitary napkin 1.

As shown in FIG. 2 and the like, the sanitary napkin 1 includes a main-body adhesive portion 32 on the non-skin-side surface of the back sheet 31, which is provided farthest on the non-skin side. The main-body adhesive portion 32 is provided to fix the sanitary napkin 1 to the wearer's clothing during wear. When the sanitary napkin 1 is viewed in the thickness direction, there is an overlapping region y32 and a non-overlapping region n32. The overlapping region y32 is a region in which a portion of the absorbent core 11 provided with the oil agent Y overlaps the main-body adhesive portion 32, and the non-overlapping region n32 is a region in which a portion of the absorbent core 11 not provided with the oil agent Y overlaps the main-body adhesive portion 32. In the sanitary napkin 1 of the present embodiment, as shown in FIG. 2, there are: a rectangular region Y11, which is a region in which the oil agent Y is provided in the absorbent core 11; and the region y32 referring to the main-body adhesive portion 32 located inside the region Y11. The main-body adhesive portion 32 provided outside the region Y11 is the non-overlapping region n32. In such a case, it is preferable that the adhesive strength of the main-body adhesive portion 32 in the overlapping region y32 is lower than the adhesive strength of the main-body adhesive portion 32 in the non-overlapping region n32. The adhesive strength is the strength of the force for fixing the sanitary napkin 1 to the clothing, and also the strength of the force required to peel the fixed sanitary napkin 1 off. The main-body adhesive portion 32 of the sanitary napkin 1 includes, on each of two lateral sides, six linear adhesive application regions (downward-right hatched portions in FIG. 2) that are intermittently arranged in the width direction, but spaces between the adhesive application regions function as adhesive portions due to impregnation of the adhesive or the like. Therefore, the main-body adhesive portion 32 of the sanitary napkin 1 is a rectangular region surrounded by the outer ends of the adhesive application regions provided on each of the two lateral sides.

The region y32 of the sanitary napkin 1 is a region in which the adhesive of the main-body adhesive portion 32 is likely to be compatible with the oil agent Y. As a result, the adhesive and the oil agent Y mix, the adhesive strength tends to

decrease. In particular, when a breathable film that is impermeable to liquids but allows gas to pass through is used as the back sheet 31, the oil agent Y provided in the absorbent core 11 is more likely to pass through the back sheet 31 and mix with the main-body adhesive portion 32. Thus, the main-body adhesive portion 32 of the sanitary napkin 1 has, in part, a portion with high adhesive strength and, in part, a portion with low adhesive strength, thereby ensuring fixing strength to the clothing while reducing the risk that the adhesive strength becomes excessively high. In the used sanitary napkin 1, in particular, the portion of the absorbent core 11 provided with the oil agent Y contains a large amount of body fluid, making such a portion likely to wrinkle and collapse. When the sanitary napkin 1 including the absorbent core 11 is removed from the clothing, the provision of the overlapping region y32 having a low adhesive strength allows the sanitary napkin 1 to be removed from the clothing with a small force. This makes the shape of the absorbent core 11 in the overlapping region y32 less likely to collapse, thereby facilitating operations such as rolling-up of the removed sanitary napkin 1 and improving disposability.

The adhesive strength of the main-body adhesive portion 32 in each region (overlapping region y32, non-overlapping region n32) can be measured by a known method.

For example, the adhesive strength can be compared by measuring the force required to peel off the main-body adhesive portion 32 in a peel test according to JIS Z 0237. Specifically, first, the overlapping region y32 and the non-overlapping region n32 of the sanitary napkin 1 are each cut into a predetermined dimension of 10 mm x 10 mm to obtain sample pieces.

Then, each sample piece is attached to a test plate, and the end portion of the sample piece is gripped and peeled off.

The load required for peeling off is measured using a load cell.

This measurement is performed a plurality of times for each sample.

The adhesive strength [N/10 mm] of the main-body adhesive portion 32 can be calculated by dividing the average value of the obtained load [N] required to peel off by the width of the sample piece (10 mm) and multiplying the result by 10.

Adhesive strength [N/10 mm] = (Load [N] $\times$ 10) / (width of the sample piece)

The adhesive strength can be compared based on the calculated adhesive strength [N/10 mm] of each sample piece. The test plate may be a SUS304 steel plate specified in JIS Z0237, or, assuming actual use, may alternatively be a horizontal plate to which a 100% cotton sheet member or a 100% polyester sheet member is fixed.

The sanitary napkin 1 is distributed, sold, stored, and the like in a folded state in which the sanitary napkin is folded at one or more fold portions F. The fold portion F is a fold line extending in the width direction. In the sanitary napkin 1, the fold portion F includes: a front fold portion F1 on the front side with respect to the center line CL in the longitudinal direction; and a back fold portion F2 on the back side with respect to the center line CL. The sanitary napkin 1 is folded at the fold portions F1 and F2 with the skin-side surfaces facing inward. The sanitary napkin 1 shown in FIG. 1 and the like, is in an unfolded state in which all fold portions F are unfolded. In the sanitary napkin 1 in the unfolded state, it is preferable that the oil agent Y is provided so as to span the fold portions F in the longitudinal direction. Specifically, as shown in FIG. 1, the region Y11 in which the oil agent Y is provided in the absorbent core 11 spans the front fold portion F1. In other words, in a plan view, the front fold portion F1 is provided at a position overlapping the region Y11. In the front fold portion F1 (fold portion F), the fiber density of the absorbent core 11 tends to be high. Since the front fold portion F1 extends in the width direction, excrement that has reached the front fold portion F1 during wear is likely to diffuse outward in the width direction along the front fold portion F1. Thus, by providing the region Y11, which is a region in which the oil agent Y is provided in the absorbent core 11 so as to span the front fold portion F1, it becomes easier for the oil agent Y in the region Y11 to promote diffusion of excrement in the longitudinal direction even when the excrement reaches the front fold portion F1 during wear. This can reduce the risk that excrement leaks to the outer side of the sanitary napkin 1 in the width direction.

Furthermore, it is preferable that the weight per unit volume of the oil agent Y provided in a portion tu on the skin side with respect to a center 11c of the absorbent core 11 in the thickness direction thereof is greater than the weight per unit volume of the oil agent Y provided in a portion tb on the non-skin side with respect to the center 11c of the absorbent core 11 in the thickness direction thereof. That is, in the thickness direction of the absorbent core 11, more oil agent Y is provided on the skin side than on the non-skin side. As a result, during wear, excrement absorbed from the skin-side surface of the sanitary napkin 1 can be more easily transferred from the skin side toward the non-skin side of the absorbent core 11.

As described above, the top sheet 21 of the sanitary napkin 1 is a sheet capable of coming into contact with the wearer's skin. It is preferable that the oil agent Y is also provided in the top sheet 21 and the top sheet 21 includes the region Y21 in which the oil agent Y is provided. As viewed in the thickness direction, it is preferable that, in the top sheet 21, the basis weight of the oil agent Y provided in a portion overlapping the central portion Tb, Wb in the longitudinal direction or the width direction is higher than the basis weight of the oil agent Y provided in a portion overlapping the one-side portion Ta, Wa in the longitudinal direction or the width direction. In the manufacturing process of the sanitary napkin 1 according to the present embodiment, in the state in which the top sheet 21 and the absorbent body 10 are stacked, the oil agent Y is applied

from the skin-surface side of the top sheet 21. Then, the oil agent Y that has permeated from the top sheet 21 remains in the absorbent core 11 and is retained therein. The oil agent Y is also provided in the top sheet 21 in addition to the absorbent core 11, and, the basis weight of the oil agent Y provided in the portion overlapping the central portion Tb, Wb of the top sheet 21 in the longitudinal direction or the width direction is higher than the basis weight of the oil agent provided in the portion overlapping the one-side portion Ta, Wa in the longitudinal direction or the width direction, thereby making it easier to promote diffusion of excrement in the portion overlapping the central portion Tb, Wb of the top sheet 21 in the longitudinal direction or the width direction. As a result, as viewed in the thickness direction, after the excrement has been allowed to diffuse to some extent in the portion overlapping the central portions Tb, Wb of the top sheet 21 in the longitudinal direction or the width direction, the excrement moves toward the non-skin side, making the excrement likely to be absorbed over a wide range of the central portion Tb, Wb of the absorbent core 11 in the longitudinal direction or the width direction. This allows the sanitary napkin 1 to more readily accelerate absorption of the excrement. Furthermore, the oil agent Y facilitates absorption of the excrement in the central portion Tb, Wb of the absorbent core 11, and also facilitates suppression of diffusion of the excrement in the one-side portion Ta, Wa of the absorbent core 11 in the longitudinal direction or the width direction.

In addition, in the case where the oil agent Y is provided in the top sheet 21 of the sanitary napkin 1, as shown in FIG. 9, it is preferable that a widthwise length W21 of the top sheet 21 is longer than a widthwise length W11 of the absorbent core 11 (W21 > W11), and that the oil agent Y is not provided in a region located outside the absorbent core 11 in the width direction. Accordingly, diffusion of excrement in the widthwise end portions of the absorbent core 11 is more readily suppressed, thereby reducing the risk that the excrement leaks from the outer side of the sanitary napkin 1 in the width direction.

In the case where the oil agent Y is provided in the top sheet 21 of the sanitary napkin 1, as shown in FIG. 9, it is preferable that the widthwise length W21 of the top sheet 21 is longer than the widthwise length W11 of the absorbent core 11 (W21 > W11). The absorbent core 11 has a core-oil-agent-present region Y11 in which the oil agent Y is provided, and a core-oil-agent-absent region N11 in which the oil agent Y is not provided. The top sheet 21 has a surface-oil-agent-present region Y21 in which the oil agent Y is provided, and a surface-oil-agent-absent region N21 in which the oil agent Y is not provided. In the thickness direction of the sanitary napkin 1, it is preferable that there are: a first region R1 in which the core-oil-agent-present region Y11 and the surface-oil-agent-present region Y21 overlap; a second region R2 in which the core-oil-agent-absent region N11 and the surface-oil-agent-present region Y21 overlap; and a third region R3 in which the surface-oil-agent-absent region N21 and the absorbent core 11 do not overlap. As shown in FIG. 9, it is preferable that the first region R1, the second region R2, and the third region R3 are arranged in order from the inner side to the outer side in the width direction. As a result, during wear, diffusion of excrement is first promoted in the first region R1 of the sanitary napkin 1, while making it easier to suppress diffusion of excrement in the second region R2 and the third region R3. Therefore, it is possible to reduce the risk that excrement leaks to the outer side of the sanitary napkin 1 in the width direction.

Furthermore, it is preferable that the core-oil-agent-present region Y11 has, in the first region R1, a core-oil-agent-high-basis-weight region Y11k in which the basis weight of the oil agent Y is higher than that in the surrounding region, and that the surface-oil-agent-present region Y21 has, in the first region R1, a surface-oil-agent-low-basis-weight region Y21u in which the basis weight of the oil agent Y is lower than that in the surrounding region. In such a case, when the sanitary napkin 1 is viewed in the thickness direction, it is more preferable that the first region R1 has, in its central portion in both the width direction and the longitudinal direction, a portion in which the core-oil-agent-high-basis-weight region Y11k and the surface-oil-agent-low-basis-weight region Y21u overlap.

The first region R1 of the top sheet 21, which is capable of coming into contact with the wearer's skin, is a portion that is likely to come into contact with the excretion opening such as the vaginal opening during wear. Since the excretion opening such as the vaginal opening is a particularly sensitive portion, the oil agent Y may cause irritation and/or have adverse effects upon a direct contact of the oil agent Y. In particular, the absorbent core 11 of the present embodiment has, in the central portion in both the longitudinal direction and width direction, the middle-high portion 11d that protrudes toward the skin side, and is therefore more likely to come into contact with the excretion opening such as the wearer's vaginal opening. On the one hand, it is preferable that the top sheet 21 includes the oil agent Y, and, particularly in the first region R1 overlapping the core-oil-agent-present region Y11 as viewed in the thickness direction, it is preferable to promote penetration and diffusion of excrement in the top sheet 21, thereby preventing excrement from flowing on the surface of the top sheet 21 (on the skin-side surface) and leaking from the sanitary napkin 1. Thus, since the top sheet 21 includes the surface-oil-agent-low-basis-weight region Y21u in the first region R1, the amount of the oil agent Y provided in a portion that is likely to directly contact the wearer's excretion opening can be reduced. This can promote penetration and diffusion of the excrement in the top sheet 21 and reduce the risk that the oil agent Y causes irritation or adverse effects to the wearer's excretion opening.

In addition, as viewed in the thickness direction, the first region R1 includes the core-oil-agent-high-basis-weight region Y11k and the surface-oil-agent-low-basis-weight region Y21u, and also includes a portion in which the core-oil-agent-high-basis-weight region Y11k and the surface-oil-agent-low-basis-weight region Y21u overlap as viewed in the

thickness direction. With this configuration, excrement is more likely to be transferred from the top sheet 21 to the non-skin side than to diffuse within the top sheet 21, thereby facilitating absorption and diffusion of excrement in the absorbent core 11 including the core-oil-agent-high-basis-weight region Y11k with a higher basis weight of the oil agent Y than that in the surrounding region. Thus, excrement is more easily drawn into the absorbent core 11, thereby reducing a risk that the excrement diffuses on the surface of the sanitary napkin 1 and leaks.

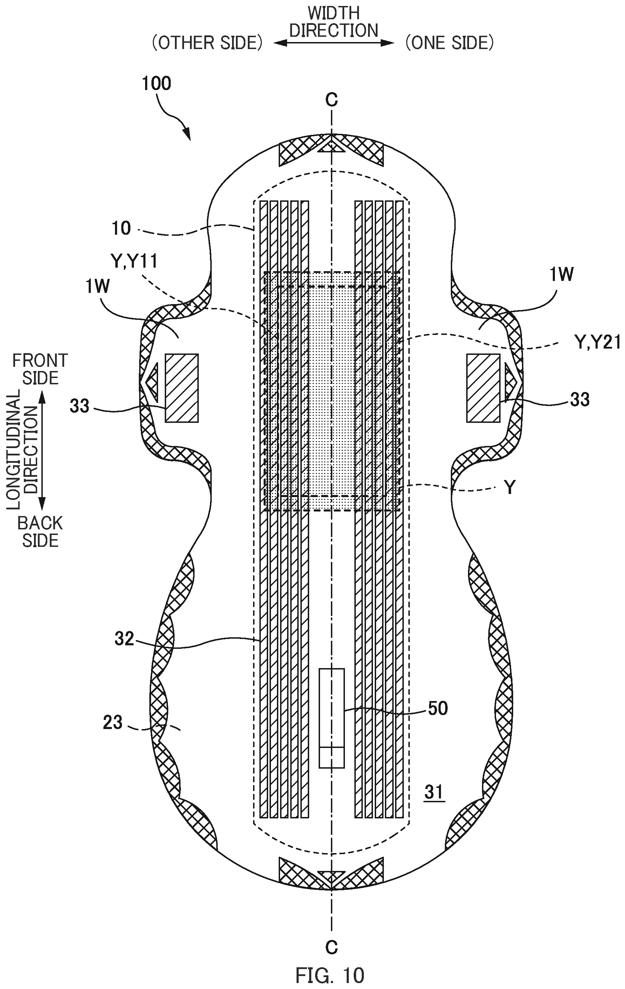

An absorbent article such as a sanitary napkin may include a post-processing tab or a post-processing tape. For example, as shown in FIG. 10, a sanitary napkin 100 may be a nighttime sanitary napkin and may include a post-processing tab 50. FIG. 10 is a plan view of the sanitary napkin 100 as viewed from the non-skin side. For the sanitary napkin 100, components having the same configuration as those of the above-described sanitary napkin 1 are given the same reference numerals, and detailed description thereof will be omitted.

The sanitary napkin 100 includes the post-processing tab 50 on the non-skin-side surface of the sanitary napkin 100, that is, on the non-skin-side surface of the back sheet 31. The post-processing tab 50 is a member used after the sanitary napkin 100 has been used. The post-processing tab 50 is a strip-shaped film member including: a fixed portion that is fixed to the back sheet 31 with an adhesive; and a free portion that is not fixed to the back sheet 31. After use of the sanitary napkin 100, the sanitary napkin 100, which has been rolled in the longitudinal direction, is wrapped by the post-processing tab 50, which is in the stretched state in the longitudinal direction, and the post-processing tab 50 is attached to the main-body adhesive portion 32 of the sanitary napkin 100, thereby making it easier to maintain the rolled state of the sanitary napkin 100.

For the post-processing tab 50, it is preferable that, when the sanitary napkin 100 is viewed in the thickness direction, the oil agent Y (region Y11) does not overlap the post-processing tab 50. In the absorbent core 11 of the used sanitary napkin 100, excrement diffuses due to the oil agent Y, thereby making the shape of the absorbent core 11 more likely to collapse. On the other hand, in the absorbent core 11, the portion that does not overlap the oil agent Y (region Y11) as viewed in the thickness direction exhibits relatively less diffusion of excrement due to the oil agent Y than the portion that overlaps the oil agent Y (region Y11). As a result, the shape of the absorbent core 11 is less likely to collapse. Thus, as viewed in the thickness direction, the post-processing tab 50 is provided in the portion that does not overlap the oil agent Y (region Y11), thereby allowing the absorbent core 11 to be easily rolled while preventing the shape of the absorbent core 11 from collapsing when the sanitary napkin 100 is rolled up for disposal after use.

The sanitary napkin 100 includes the post-processing tab 50, but the present disclosure is not limited thereto. For example, in the case where an absorbent article is a disposable diaper or shorts-type sanitary napkin, the absorbent article may include a post-processing tape for maintaining the used absorbent article in a rolled state. The post-processing tape is a film member in which a longitudinal one-side end portion of the tape is fixed to the absorbent article and in which an adhesive portion is provided in a longitudinal other-side end portion. After use, the absorbent article in a rolled state is wrapped by the post-processing tape and fastened by the adhesive portion at the longitudinal other-side end portion of the post-processing tape, thereby more readily maintaining the rolled state of the absorbent article. Similarly to the post-processing tab 50, in the absorbent article including such a post-processing tape, it is preferable that the post-processing tape is provided at a position that does not overlap the oil agent Y provided in the absorbent core 11 as viewed in the thickness direction.

Other Embodiments

Although the embodiment of the present disclosure has been described above, the above embodiment is simply to facilitate understanding of the present disclosure and is not in any way to be construed as limiting the present disclosure. Further, the present disclosure may variously be changed or altered without departing from its gist and encompass equivalents thereof.

REFERENCE SIGNS LIST

1: sanitary napkin (absorbent article)
1W: wing portion
10: absorbent body
11: absorbent core, 11d: middle-high portion
12: core-wrapping sheet
12a: skin-side portion (core-wrapping sheet), 12b: non-skin-side portion (core-wrapping sheet)
14: central compressed portion, 15: absorbent-body compressed portion
21: top sheet (top-face sheet)
22: second sheet

23: side sheet, 24: reinforcing sheet
31: back sheet (non-skin-side sheet)
32: main-body adhesive portion (adhesive portion), 33: wing adhesive portion
CA: crotch region
ee: circular compressed portion, EL: line-like compressed portion
Ta: front-side portion (one-side portion), Tb: central portion, Tc: back-side portion (other-side portion)
Wa: left-side portion (one-side portion), Wb: central portion, Wc: right-side portion (other-side portion)
Y: oil agent
Y11: region (core-oil-agent-present region), Y11k: core-oil-agent-high-basis-weight region
N11: core-oil-agent-absent region
Y21: region (surface-oil-agent-present region), Y21u: surface-oil-agent-low-basis-weight region
N21: surface-oil-agent-absent region
R1: first region, R2: second region, R3: third region

## Claims

**1.** An absorbent article having a longitudinal direction, a width direction, and a thickness direction that are orthogonal to one another, the absorbent article comprising:

an absorbent core that has pulverized fibers,

the pulverized fibers including hardwood fibers that are made from hardwood,
an oil agent being provided in the absorbent core, the oil agent promoting diffusion of excrement,

in either the longitudinal direction or the width direction,
when the absorbent core is divided into three equal portions,
the three equal portions being a one-side portion, a central portion, and an other-side portion, in order from one side,
a basis weight of the oil agent provided in the central portion being higher than a basis weight of the oil agent provided in the one-side portion.

**2.** The absorbent article according to claim 1, wherein

the pulverized fibers include softwood fibers that are made from softwood, and
a total weight of the hardwood fibers is greater than a total weight of the softwood fibers.

**3.** The absorbent article according to claim 1 or 2, wherein

the absorbent article further comprises a top-face sheet capable of coming into contact with a skin of a wearer,
the oil agent is provided in the top-face sheet, and
as viewed in the thickness direction, in the top-face sheet, a basis weight of the oil agent provided in a portion overlapping the central portion is higher than a basis weight of the oil agent provided in a portion overlapping the one-side portion.

**4.** The absorbent article according to claim 1 or 2, wherein
the oil agent is an amphiphilic substance.

**5.** The absorbent article according to claim 1 or 2, wherein

a core-wrapping sheet is provided on a skin side with respect to the absorbent core, and
an adhesive is not provided between a skin-side surface of the absorbent core and a non-skin-side surface of the core-wrapping sheet.

**6.** The absorbent article according to claim 1 or 2, wherein

a core-wrapping sheet is provided on a non-skin side with respect to the absorbent core, and
an adhesive is not provided between a non-skin-side surface of the absorbent core and a skin-side surface of the

core-wrapping sheet.

7. The absorbent article according to claim 1 or 2, wherein

a post-processing tab or a post-processing tape is provided on a non-skin-side surface of the absorbent article, and
as viewed in the thickness direction, the oil agent does not overlap the post-processing tab or the post-processing tape.

8. The absorbent article according to claim 1 or 2, wherein

a non-skin-side sheet is provided farthest on a non-skin side of the absorbent article,
an adhesive portion is provided on a non-skin-side surface of the non-skin-side sheet,
as viewed in the thickness direction, there is an overlapping region and a non-overlapping region,

the overlapping region being a region in which a portion provided with the oil agent overlaps the adhesive portion,
the non-overlapping region being a region in which a portion not provided with the oil agent overlaps the adhesive portion, and

an adhesive strength of the adhesive portion in the overlapping region is lower than an adhesive strength of the adhesive portion in the non-overlapping region.

9. The absorbent article according to claim 1 or 2, wherein

the absorbent core includes a core-wrapping sheet on a skin side of the absorbent core, and
an average fiber length of fibers of the core-wrapping sheet is equal to or greater than an average fiber length of the pulverized fibers of the absorbent core.

10. The absorbent article according to claim 1 or 2, wherein
in the longitudinal direction and the width direction, a basis weight of the oil agent provided in the central portion is higher than a basis weight of the oil agent provided in the one-side portion.

11. The absorbent article according to claim 10, wherein
the oil agent is not provided in an outer end portion of the one-side portion.

12. The absorbent article according to claim 1 or 2, wherein

the absorbent article further comprises a top-face sheet capable of coming into contact with a skin of a wearer,
the oil agent is provided in the top-face sheet,
a widthwise length of the top-face sheet is longer than a widthwise length of the absorbent core,
the absorbent core has a core-oil-agent-present region and a core-oil-agent-absent region,

the core-oil-agent-present region being a region in which the oil agent is provided,
the core-oil-agent-absent region being a region in which the oil agent is not provided,

the top-face sheet has a surface-oil-agent-present region and a surface-oil-agent-absent region,

the surface-oil-agent-present region being a region in which the oil agent is provided,
the surface-oil-agent-absent region being a region in which the oil agent is not provided,

in the thickness direction,
there are a first region, a second region, and a third region,

the first region being a region in which the core-oil-agent-present region and the surface-oil-agent-present region overlap,
the second region being a region in which the core-oil-agent-absent region and the surface-oil-agent-present region overlap,

the third region being a region in which the surface-oil-agent-absent region and the absorbent core do not overlap, and

the first region, the second region, and the third region are arranged in order from an inner side to an outer side in the width direction.

**13.** The absorbent article according to claim 12, wherein

the core-oil-agent-present region has, in the first region, a core-oil-agent-high-basis-weight region in which a basis weight of the oil agent is higher than that in a surrounding region,
the surface-oil-agent-present region has, in the first region, a surface-oil-agent-low-basis-weight region in which the basis weight of the oil agent is lower than that in a surrounding region, and
as viewed in the thickness direction, the first region has, in a central portion in both the width direction and the longitudinal direction, a portion in which the core-oil-agent-high-basis-weight region and the surface-oil-agent-low-basis-weight region overlap.

**14.** The absorbent article according to claim 1 or 2, wherein

the absorbent article further comprises a top-face sheet capable of coming into contact with a skin of a wearer,
the oil agent is provided in the top-face sheet,
a widthwise length of the top-face sheet is longer than a widthwise length of the absorbent core, and
the oil agent is not provided in a region located outside the absorbent core in the width direction.

**15.** The absorbent article according to claim 1 or 2, wherein

the absorbent article further comprises a compressed portion in which the absorbent core is compressed in the thickness direction,
the compressed portion includes a back compressed portion on a back side with respect to a center of the absorbent article in the longitudinal direction, and
a back end of the oil agent is provided on a front side with respect to a front end of the back compressed portion.

**16.** The absorbent article according to claim 1 or 2, wherein

a weight per unit volume of the oil agent provided on a skin side with respect to a center of the absorbent core in the thickness direction
is greater than
a weight per unit volume of the oil agent provided on a non-skin side with respect to the center of the absorbent core in the thickness direction.

**17.** The absorbent article according to claim 1 or 2, wherein

the absorbent article further comprises a fold portion extending in the width direction, and
in the longitudinal direction, the oil agent is provided so as to span the fold portion.

WIDTH DIRECTION
(ONE SIDE)
(OTHER SIDE)
C
1
10
EL,ELa
F1
1W
Y,Y11
Y,Y21
1W
FRONT SIDE
33
33
CA
A
A
LONGITUDINAL DIRECTION
CL
ee
ee
EL
EL,ELc
BACK SIDE
F2
11d
EL,ELb
EL,ELb
23
23
EL,ELb
EL,ELb
21
C

FIG. 1

WIDTH
DIRECTION
(OTHER SIDE)
(ONE SIDE)
1
C
10
Y,Y11
Y,Y21
1W
1W
33
33
FRONT
SIDE
LONGITUDINAL
DIRECTION
BACK
SIDE
CL
y32
y32
32
32
n32
n32
23
23
31
C

FIG. 2

1
WIDTH
DIRECTION
(ONE SIDE)
(OTHER SIDE)
ELx,EL
11
21
22
ELy,EL
12
23
24
33
32
31
SKIN SIDE
THICKNESS
DIRECTION
NON-SKIN
SIDE

FIG. 3

WIDTH
DIRECTION
(ONE SIDE)
(OTHER SIDE)
C
J
11
11f
11d
FRONT
SIDE
LONGITUDINAL
DIRECTION
BACK
SIDE
K
K
CL
J
C
THICKNESS
DIRECTION
SKIN SIDE
NON-SKIN
SIDE
11
FRONT
SIDE
LONGITUDINAL
DIRECTION
BACK
SIDE
CL
11d

FIG. 4A

FIG. 4B

WIDTH DIRECTION
(ONE SIDE)
(OTHER SIDE)
C
C
tu
11c
tb
11
SKIN SIDE
THICKNESS DIRECTION
NON-SKIN SIDE

FIG. 5

WIDTH
DIRECTION
(ONE SIDE)
(OTHER SIDE)
10
C
12a(12)
SKIN SIDE
THICKNESS
DIRECTION
NON-SKIN
SIDE
12
12ea
12eb
11
12b(12)
C

FIG. 6

WIDTH
DIRECTION
(ONE SIDE)
(OTHER SIDE)
C
14
15
10
12
11
12ea
12eb
FRONT
SIDE
LONGITUDINAL
DIRECTION
BACK
SIDE
CL
C

FIG. 7

WIDTH
DIRECTION
(ONE SIDE)
(OTHER SIDE)
1
C
10
Ta
1W
Y21
1W
33
(FRONT SIDE)
ONE SIDE
Y21u
Y11,R1
D
D
Y11k
33
LONGITUDINAL
DIRECTION
CL
Tb
T11
OTHER SIDE
(BACK SIDE)
EL
EL
11d
23
23
Tc
21
C
Wa
Wb
Wc
W11

FIG. 8

WIDTH DIRECTION
(ONE SIDE)
(OTHER SIDE)
W21
N21
Y21
N21
1
Y21u
C
21
23
23
SKIN SIDE
THICKNESS DIRECTION
NON-SKIN SIDE
10(11)
31
33
32
C
33
Y11k
Wa
Wb
Wc
W11
N11
Y11
N11
R1
R3
R2
R2
R3

FIG. 9

WIDTH DIRECTION
(OTHER SIDE)
(ONE SIDE)
100
C
10
Y,Y11
1W
1W
Y,Y21
FRONT SIDE
LONGITUDINAL DIRECTION
BACK SIDE
33
33
Y
32
50
23
31
C

FIG. 10

## INTERNATIONAL SEARCH REPORT

International application No.
**PCT/JP2024/038160**

**A. CLASSIFICATION OF SUBJECT MATTER**

***A61F 13/535***(2006.01)i; ***A61F 13/53***(2006.01)i; ***A61F 13/56***(2006.01)i; ***A61F 13/511***(2006.01)i; ***A61F 13/536***(2006.01)i; ***A61F 13/539***(2006.01)i
FI: A61F13/535 100; A61F13/511 200; A61F13/53 100; A61F13/53 300; A61F13/536; A61F13/539; A61F13/56 110

According to International Patent Classification (IPC) or to both national classification and IPC

**B. FIELDS SEARCHED**

Minimum documentation searched (classification system followed by classification symbols)

A61F13/15-13/84

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2021-83539 A (UNI-CHARM CO., LTD.) 03 June 2021 (2021-06-03) | 1-17 |
| A | JP 2014-113273 A (KAO CORPORATION) 26 June 2014 (2014-06-26) | 1-17 |
| A | JP 2018-164732 A (SDP GLOBAL CO., LTD.) 25 October 2018 (2018-10-25) | 1-17 |
| A | JP 2012-143534 A (KAO CORPORATION) 02 August 2012 (2012-08-02) | 1-17 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/038160**

| Patent document cited in search report | Publication date (day/month/year) | Patent family member(s) | Publication date (day/month/year) |
|---|---|---|---|
| JP 2021-83539 A | 03 June 2021 | (Family: none) | |
| JP 2014-113273 A | 26 June 2014 | (Family: none) | |
| JP 2018-164732 A | 25 October 2018 | (Family: none) | |
| JP 2012-143534 A | 02 August 2012 | WO 2012/086265 A1 | |
| | | CN 103260570 B | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013179982 A